# EUROPEAN PATENT APPLICATION

(11) **EP 0 747 478 A1**
(43) Date of publication of application: **11.12.1996**
(21) Application number: 95941844.3
(22) Date of filing: 22.12.1995
(51) Int. Cl.: C12N 9/90, C12N 15/00

(54) **DNA ENCODING MALEATE ISOMERASE AND USE OF THE SAME**

(30) Priority: 22.12.1994 JP 320112/94; 22.06.1995 JP 179498/95
(71) Applicant: MITSUBISHI CHEMICAL CORPORATION, Chiyoda-ku, Tokyo 100 (JP)
(72) Inventor: HATAKEYAMA, Kazuhisa, Mitsubishi Chemical Corp., Inashiki-gun, Ibaraki 300-03 (JP); ASAI, Yoko, Mitsubishi Chemical Corp., Inashiki-gun, Ibaraki 300-03 (JP); KATO, Yukie, Mitsubishi Chemical Corp., Inashiki-gun, Ibaraki 300-03 (JP); UCHIDA, Yasukazu, Mitsubishi Chemical Corp., Inashiki-gun, Ibaraki 300-03 (JP); KOBAYASHI, Miki, Mitsubishi Chemical Corp., Inashiki-gun, Ibaraki 300-03 (JP); YUKAWA, Hideaki, Mitsubishi Chemical Corp., Inashiki-gun, Ibaraki 300-03 (JP)
(74) Representative: Kolb, Helga, Dr. Dipl.-Chem.
(86) International application number: JP9502631
(87) International publication number: WO9619571

(57) **Abstract**

Fumaric acid is produced by isomerizing maleic acid into fumaric acid by using microbial cells of a transformant strain of Escherichia coli harboring a maleate isomerase gene originating from Alcaligenes faecalis, Bacillus stearothermophilus, Bacillus brevis, or Serratia marcescens, or maleate isomerase collected from the transformant.

## Description

### Technical Field

The present invention relates to DNA coding for maleate isomerase and utilization thereof. In particular, the present invention relates to DNA coding for an enzyme having specified partial amino acid sequences and having an activity to isomerize maleic acid and produce fumaric acid, a recombinant vector containing the DNA ligated therewith, a transformant harboring the DNA, a method for producing the enzyme by using the transformant, and a method for producing fumaric acid.

Fumaric acid, which is an isomerized product obtained from maleic acid, is a useful organic acid utilizable as a raw material for various fine chemicals such as aspartic acid and malic acid.

### Background Art

Chemical methods have been principally proposed as methods for producing fumaric acid by isomerizing maleic acid (United States Patent Nos. 2,816,923, 2,955,136, and 2,332,992). However, these methods involve, for example, the following problems. Firstly, the ratio of conversion into fumaric acid is restricted by reaction equilibrium. Secondly, maleic acid or fumaric acid is deteriorated because these methods involve a reaction at a high temperature. Finally, the yield is decreased due to generation of by-products.

On the other hand, as for isomerization of maleic acid by an enzyme, it is known that maleate isomerase (maleate cis-trans isomerase) isomerizes maleic acid into fumaric acid. Known microorganisms which produce maleate isomerase include, for example, Pseudomonas sp. [K. Otsuka, Agric. Biol. Chem., Vol. 25, No. 9, p. 726 (1961)], Alcaligenes faecalis IB-14 [Takamura, Agric. Biol. Chem., Vol. 33, p. 718 (1969)], Pseudomonas fluorescens ATCC 23728 [Scher, J. Biol. Chem., Vol. 244, p. 1878 (1969)], and Serratia marcescens IFO 3736 [T. Kimura, Agric. Biol. Chem., Vol. 50, No. 1, p. 82 (1986)]. However, as for maleate isomerases produced by these microorganisms, only a part of enzymatic properties have been reported. Details of these enzymes have not been clarified, such as correct molecular weights, subunit structures, entire amino acid sequences, and entire nucleotide sequences of DNA's. No investigation has been made for maleate isomerase based on a viewpoint of industrial application at all, as far as the present inventors know.

If a gene coding for maleate isomerase is obtained, and the gene is introduced into a microorganism by using an appropriate vector to obtain a transformant harboring the gene in multiple copies in cells, then it becomes possible to conspicuously increase the microbial catalyzing ability as compared with conventional methods.

### Disclosure of the Invention

An object of the present invention is to provide DNA coding for maleate isomerase originating from a microorganism, and a method for producing fumaric acid by using the DNA.

As a result of diligent investigations in order to isolate DNA fragments of genes each coding for maleate isomerase from microorganisms belonging to the genera Alcaligenes, Bacillus, and Serratia, the present inventors have succeeded in isolating a DNA fragment of such a gene, incorporating the fragment (especially a DNA fragment comprising, in its nucleotide sequence of DNA, sequences corresponding to amino acid sequences depicted in SEQ ID NOs: 1 and 2) into a vector to obtain a recombinant vector, transforming a microorganism with the recombinant vector to prepare a transformant having an increased activity of maleate isomerase, and using the transformant to efficiently produce fumaric acid and various derivatives thereof by using fumaric acid as a raw material. Thus the present invention has been completed.

According to the present invention, there are provided:
(a) DNA coding for maleate isomerase wherein the maleate isomerase comprises at least an amino acid sequence shown in SEQ ID NO: 1 and an amino acid sequence shown in SEQ ID NO: 2, optionally having substitution, deletion, and insertion of one or more amino acid residues which do not substantially deteriorate an activity to isomerize maleic acid and produce fumaric acid;
(b) a recombinant vector comprising the DNA described in the foregoing item (a) ligated with a vector;
(c) a transformant obtained by transformation by introducing the DNA described in the foregoing item (a);
(d) a method for producing maleate isomerase comprising the steps of cultivating the transformant described in the foregoing item (c) in a medium, and collecting maleate isomerase from a culture of the transformant; and
(e) a method for producing fumaric acid comprising the steps of adding, to an aqueous solution containing maleic acid, the transformant described in the foregoing item (c), or a crude fraction or a purified enzyme of maleate isomerase collected from a culture of the transformant, and isomerizing maleic acid to produce fumaric acid.

The present invention will be explained in detail below.

The "maleate isomerase (maleate cis-trans isomerase; EC 5.2.1.1)" of the present invention means an enzyme which catalyzes a reaction to isomerize maleic acid (cis form) and produce fumaric acid (trans form). In this specification, DNA coding for maleate isomerase is conveniently referred to as "maleate isomerase gene", if necessary.

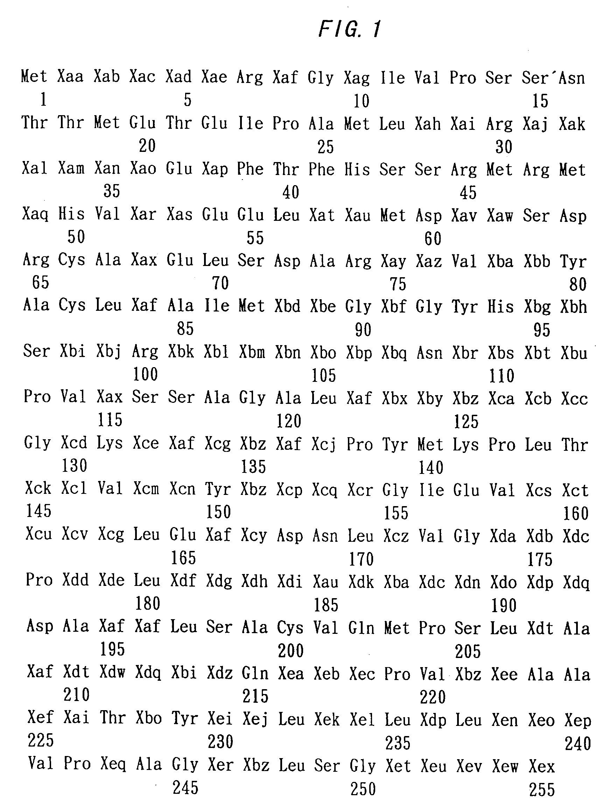

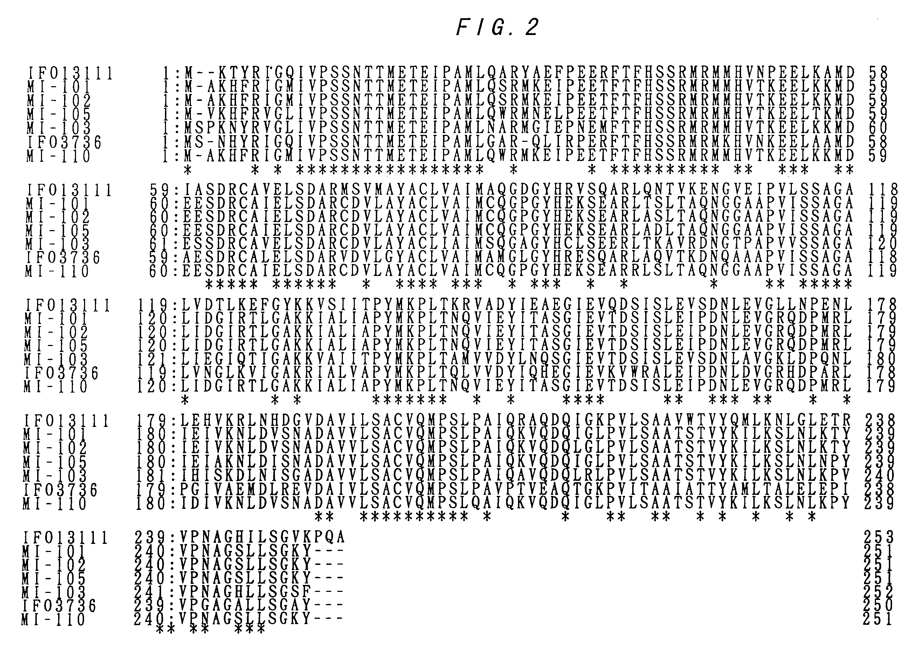

The maleate isomerase gene of the present invention has, as partial nucleotide sequences, nucleotide sequences capable of coding for amino acid sequences shown in SEQ ID NOs: 1 and 2, which is exemplified by DNA coding for an amino acid sequence shown in SEQ ID NO: 3. In relation to the amino acid sequence shown in SEQ ID NO: 3, details of amino acids represented by Xaa are shown in Fig. 1 and Table I described below. The nucleotide sequence of DNA of the present invention specifically includes those shown in SEQ ID NOs: 4, 11, 18, 25, 32, 39, and 46. Amino acid sequences encoded by these nucleotide sequences are shown in SEQ ID NOs: 5, 12, 19, 26, 33, 40, and 47. Fig. 2 shows comparison among these amino acid sequences. In Fig. 2, IFO 13111, MI-101, MI-102, MI-105, MI-103, IFO 3736, and MI-110 indicate strains of Alcaligenes faecalis IFO 13111, Bacillus stearothermophilus MI-101 and MI-102, Bacillus brevis MI-103, Bacillus stearothermophilus MI-105 and MI-110, and Serratia marcescens IFO 3736 respectively from which the respective maleate isomerase genes originate.

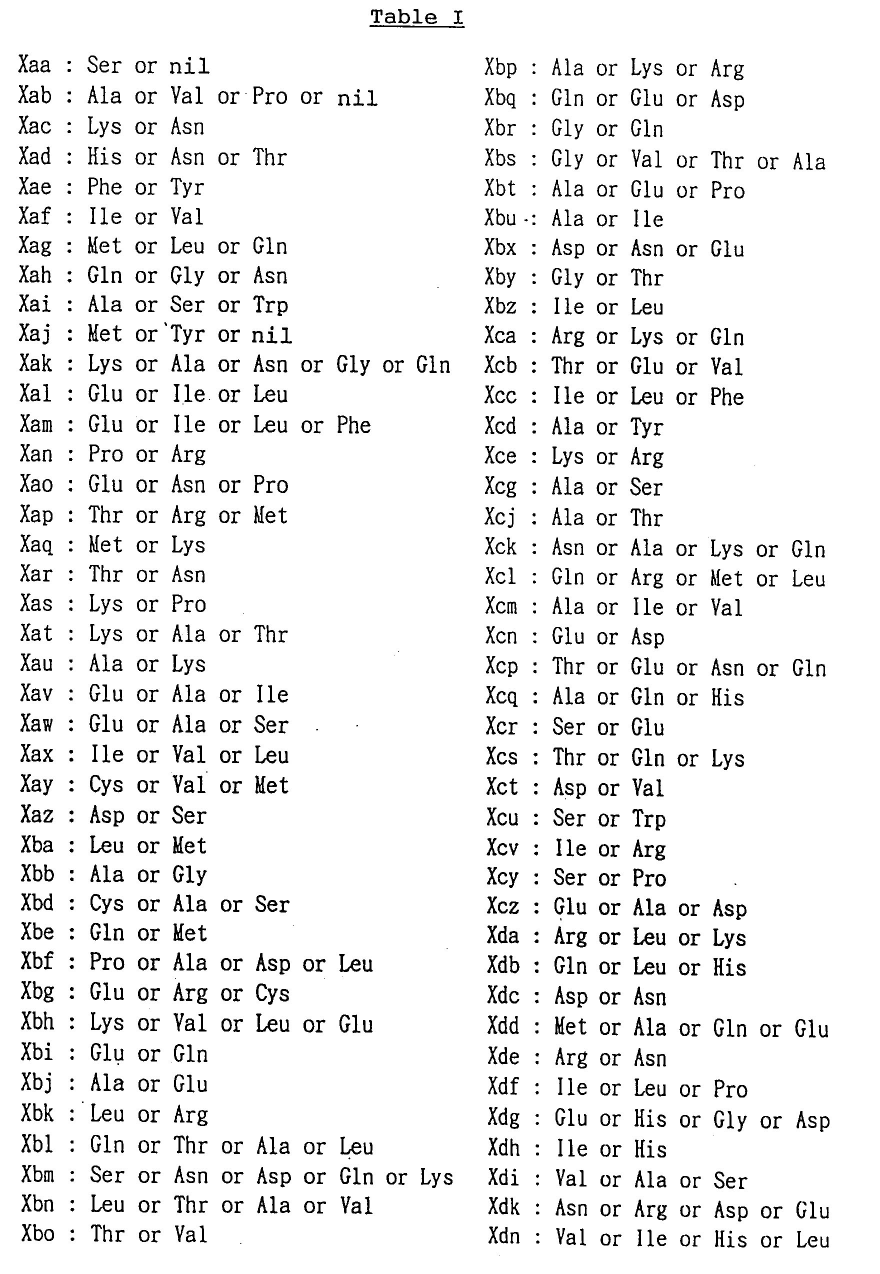

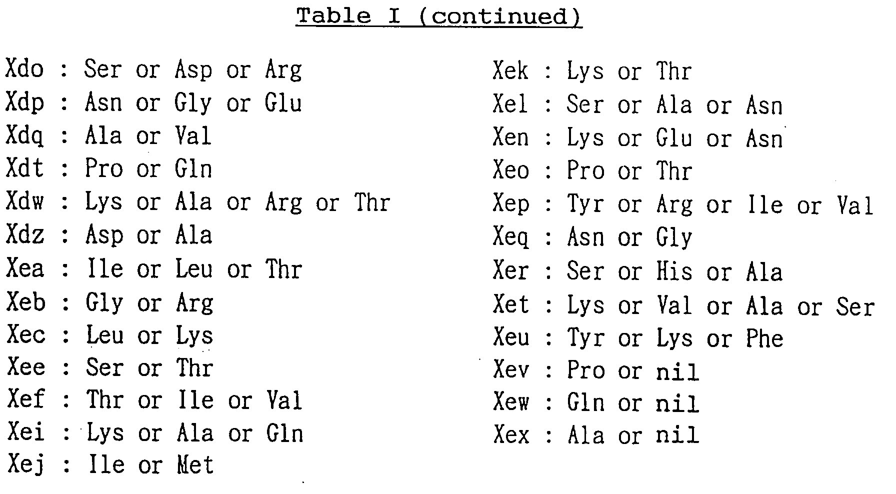

The DNA of the present invention is not limited to the sequences described above. Any of DNA coding for maleate isomerase having substitution, deletion, and insertion of one or more amino acid residues in the amino acid sequence described above, and DNA coding for maleate isomerase hybridizable to such DNA is included in the DNA of the gene of the present invention provided that the activity to isomerize maleic acid and produce fumaric acid is not substantially deteriorated.

The maleate isomerase has been suggested to be an SH enzyme in which an SH residue of cysteine contained in the enzyme protein relates to an active site according to the following results. Firstly, the storage stability is increased, or the enzyme activity is enhanced by the addition of dithiothreitol. Secondly, the enzyme activity is inhibited by an SH reagent such as PCMB (p-chloromercuribenzoate) and IAA (monoiodo acetate) and an oxidizing reagent such as sodium metaperiodate. Finally, the inhibition on the enzyme by Hg²⁺ and PCMB is desensitized by the addition of an SH compound such as dithiothreitol, 2-mercaptoethanol, L-cysteine, and glutathione [K. Otsuka, Agric. Biol. Chem., Vol. 25, p. 726, 1961; Y. Takamura et al., Agric. Biol. Chem., Vol. 33, p. 718, 1969]. In the present invention, it is especially important to possess, in the gene, sequences shown in SEQ ID NOs: 1 and 2.

Three cysteine residues are present at 64th, 80th, and 198th positions in an amino acid sequence of maleate isomerase originating from Alcaligenes faecalis IFO 13111 shown in SEQ ID NO: 5. In order to clarify which cysteine residue of the three cysteine residues of this maleate isomerase is important for the maleate isomerase activity, the present inventors have substituted the three cysteine residues with other amino acid residues such as cysteine respectively by means of site-directed mutagenesis, and investigated maleate isomerase activities of obtained mutant enzymes. As a result, a maleate isomerase obtained by substituting 64th cysteine with serine had an activity conserved approximately equivalently to that of the parent strain. However, as for those obtained by substituting 80th or 198th cysteine with serine, the maleate isomerase disappeared approximately completely in any of the both cases. According to the result, it has been demonstrated that the 80th and 198th cysteine residues are important for the maleate isomerase activity of this enzyme.

As described above, the substitution, deletion, and insertion of one or more amino acid residues which do not substantially deteriorate the activity to isomerize maleic acid and produce fumaric acid are specifically exemplified by the substitution of the 64th cysteine with serine. It is expected that the activity to isomerize maleic acid and produce fumaric acid is not substantially deteriorated even when the 64th cysteine residue is substituted with another amino acid having no SH group. The maleate isomerase encoded by the DNA of the present invention may involve substitution, deletion, and insertion of one or more amino acid residues at another position or other positions provided that the maleate isomerase activity is not affected. The position of such an amino acid residue and the mode of mutation may be easily selected by those skilled in the art. Maleate isomerase involving substitution, deletion, and insertion of one or more amino acid residues which do not substantially affect the enzyme activity may be obtained from a natural mutant strain.

Preparation of DNA coding for maleate isomerase having substitution, deletion, and insertion of one or more amino acid residues is achieved by an ordinary mutation operation including, for example, a method based on the use of a mutating agent and a method of site-directed mutagenesis. Confirmation for whether or not maleate isomerase encoded by obtained DNA has the enzyme activity may be performed by measuring the enzyme activity in accordance with, for example, a method of Otsuka et al. [Agric. Biol. Chem., Vol. 25, p. 726 (1961)] described later on.

The technique for site-directed mutagenesis includes various techniques [for example, R. Higuchi et al., "Recombinant PCR" in "PCR Protocols: A Guide to Methods and Applications", p. 177, Academic Press, 1990; and Sambrook, Fritsche and Maniatis, "Molecular Cloning", Chapter 15, "Site-directed Mutagenesis of Cloned DNA", Cold Spring Harbor Laboratory Press, 1989]. Any technique may be used provided that the technique permits introduction of mutation in a site-directed manner.

The maleate isomerase gene of the present invention has its nucleotide sequence determined by the present invention, and hence it can be synthesized on the basis of the determined sequence. However, the gene is usually cloned from a microorganism. The microorganism to serve as a source of the gene is not specifically limited provided that the microorganism has the maleate isomerase activity. Specifically, the microorganism includes, for example, Alcaligenes faecalis IFO 13111, IFO 12669, IAM 1473, and IAM 12588, Alcaligenes denitrificans IAM 12370, Alcaligenes xylosoxidans IAM 12686, Alcaligenes eutrophus IAM 12305, Pseudomonas fluorescens ATCC 23723, Flavobacterium lutescence IAM 1667, Arthrobacter globiformis IAM 12102, Arthrobacter pascens IAM 12343, Arthrobacter sulfureus IAM 1488, Arthrobacter ureafaciens IAM 1637, Serratia marcescens IFO 3736 strain, Bacillus stearothermophilus MI-101 (FERM BP-5160), MI-102 (FERM BP-5161), MI-105 (FERM BP-5164), and MI-110 (FERM BP-5339), and Bacillus brevis MI-103 (FERM BP-5162).

Among them, those preferably used include, for example, Alcaligenes faecalis IFO 13111 strain, Serratia marcescens IFO 3736 strain, Bacillus stearothermophilus MI-101, MI-102, MI-105, and MI-110, and Bacillus brevis MI-103.

The microbial strains are obtainable, for example, from Institute for Fermentation, Osaka (IFO), IAM Culture Collection of Center for Cellular and Molecular Research of Institute of Molecular and Cellular Biosciences of The University of Tokyo, and American Type Culture Collection (ATCC). The microbial strains belonging to the genus Bacillus described above are strains isolated by the present inventors as explained in Example 1 described later on, and they have been deposited in National Institute of Bioscience and Human Technology of Agency of Industrial Science and Technology of Ministry of International Trade and Industry.

Examples of a method for obtaining the maleate isomerase gene from the source microorganisms, a method for producing maleate isomerase as a product of the gene, and a method for producing fumaric acid by isomerizing maleic acid will be explained below.

The maleate isomerase gene usually exists on chromosome of the source microorganism as described above, which can be separated and obtained from a DNA bank originating from the chromosome in accordance with a hybridization method described below.

### (1) Purification of maleate isomerase, determination of partial amino acid sequence thereof, and preparation of DNA probe based thereon:

Any one of known methods concerning purification of enzymes may be used as a method for extracting and purifying maleate isomerase from microbial cells of strains such as Alcaligenes faecalis IFO 13111, Bacillus stearothermophilus MI-101 (FERM BP-5160), MI-102 (FERM BP-5161), MI-105 (FERM BP-5164), and MI-110 (FERM BP-5339), Bacillus brevis MI-103 (FERM BP-5162), and Serratia marcescens IFO 3736.

Those usable as a method for disrupting microbial cells include, for example, mechanical disrupting methods by using, for example, ultrasonic disruption, French press, or homogenizer; and enzymatic disrupting methods by using, for example, lysozyme. Maleate isomerase can be obtained as a crude enzyme solution by separating a soluble fraction from a disrupted preparation of the microbial cells.

A method for purifying maleate isomerase from the obtained crude enzyme solution can be usually carried out by combining methods of (a) separation by precipitation, for example, ammonium sulfate precipitation, (b) separation by chromatography, for example, ion exchange chromatography, affinity adsorption chromatography, gel filtration chromatography, and (c) separation by electrophoresis. An example thereof will be described in detail in Example 2 (1)-1.

Fractions containing maleate isomerase in the purification steps as described above can be detected by measuring the maleate isomerase activity on the basis of the decrease in substrate maleic acid as the decrease in absorbance at 240 nm caused by the maleate isomerase activity in the presence of swine heart fumarase (produced by Boehringer Mannheim) in accordance with a method of Otsuka et al. [Agric. Biol. Chem., Vol. 25, p. 726 (1961)].

Purified maleate isomerase is subjected to polyacrylamide gel electrophoresis in accordance with a method of Davis [B. J. Davis, Ann. N. Y. Acad. Sci., Vol. 121, p. 404 (1964)] or a method of Laemmli [U. K. Laemmli, Nature, Vol. 227, p. 680 (1970)]. The enzyme protein is stained by using, for example, a Coomassie Blue dyestuff solution [composition: 0.2 % (w/v) Coomassie Brilliant Blue R250, 40 % (v/v) methanol, 10 % (v/v) acetic acid] or a silver staining kit (produced by Wako Pure Chemical Industries). Thus it is possible to know the purity achieved by purification, the molecular weight of maleate isomerase, and other properties.

A partial amino acid sequence of purified maleate isomerase can be determined as follows. At first, the enzyme is separated into constitutional subunits of the enzyme. After that, an amino acid sequence from the N-terminal of each of the subunits, or an amino acid sequence from the N-terminal of a peptide fragment obtained by digesting each of the subunits with an appropriate proteolytic enzyme is determined by using an amino acid sequencer (produced by Applied Biosystems, 473A type) based on the Edman degradation method.

Next, a probe having a nucleotide sequence deduced from the obtained amino acid sequence is synthesized by using a DNA synthesizer (produced by Applied Biosystems, 394 type).

Examples of partial amino acid sequences of maleate isomerase obtained as described above, and examples of nucleotide sequences of probe DNA's deduced from the amino acid sequences are shown in Table 1 by using corresponding SEQ ID NOs.

**Table 1**

| | Partial amino acid sequence | Probe DNA |
|---|---|---|
| Alcaligenes faecalis IFO 13111 | SEQ ID NO: 6 | SEQ ID NO: 7, 8 |
| Bacillus stearothermophilus MI-101 | 13 | 14, 15 |
| Bacillus stearothermophilus MI-102 | 20 | 21, 22 |
| Bacillus brevis MI-103 | 27 | 28, 29 |
| Bacillus stearothermophilus MI-105 | 34 | 35, 36 |
| Bacillus stearothermophilus MI-110 | 41 | 42, 43 |
| Serratia marcescens IFO 3736 | 48 | 49, 50 |

### (2) Isolation of chromosomal DNA fragment containing maleate isomerase gene:

Any one of known methods concerning isolation of genes may be used as a method for isolating chromosomal DNA containing a maleate isomerase gene from microbial cells of strains such as Alcaligenes faecalis IFO 13111, Bacillus stearothermophilus MI-101 (FERM BP-5160), MI-102 (FERM BP-5161), MI-105 (FERM BP-5164), and MI-110 (FERM BP-5339), Bacillus brevis MI-103 (FERM BP-5162), and Serratia marcescens IFO 3736. An example of the method will be explained below.

### (A) Preparation of chromosomal DNA library:

In order to extract chromosomal DNA from the microbial strain as described above, it is possible to use microbial cells of the microbial strain cultivated in an appropriate medium. However, it is also possible to use a stock sample frozen and stored after collecting cultivated microbial cells.

Obtained chromosomal DNA is partially degraded with an appropriate restriction enzyme, for example, XhoI, Sau3AI, and TaqI to prepare a chromosomal DNA library by using a host-vector system such as those for Escherichia coli. Specifically, usable vectors include, for example, lambda phage vectors such as λFIXII (produced by Toyobo), and plasmid vectors such as pUC118 (produce by Takara Shuzo), pBR322 (produce by Takara Shuzo), and cosmid pWE15 (produced by Stratagene).

Various DNA fragments obtained by the partial degradation described above may be inserted into the vector, for example, into the phage vector λFIXII (produced by Toyobo) by ligating the vector having been cleaved with an appropriate restriction enzyme such as XhoI, Sau3AI, and TaqI with the partially degraded DNA fragments by using T4 DNA ligase. Thus the chromosomal DNA library is obtained.

### (B) Selection of vector containing maleate isomerase gene:

In order to select a vector containing a maleate isomerase gene from the chromosomal DNA library prepared in the item (A) described above, a host microorganism, for example, Escherichia coli may be subjected to transduction or transformation by using the chromosomal DNA library, and a clone which harbors the maleate isomerase gene may be selected from obtained transductants or transformants in accordance with an appropriate means.

Specifically, Escherichia coli, for example P2392 strain [Ausubel et al., Nucleic Acid Res., Vol. 7, p. 1513 (1979)] is infected with the phage vector described above, which is overlaid on an agar medium to form plaques.

After that, phage DNA in the plaques is transferred onto a nitrocellulose membrane to immobilize the phage DNA on the nitrocellulose membrane, followed by plaque hybridization ["Molecular Cloning", Cold Spring Harbor Laboratory Press (1989)] by using the probe prepared in the foregoing item (1), for example, the synthetic oligonucleotide having the nucleotide sequence shown in Table 1.

Thus a transductant, which contains a phage vector having the maleate isomerase gene originating from the chromosomal DNA of the objective microbial strain, can be detected and selected.

Alternatively, when a chromosomal DNA library is prepared by using the plasmid vector described above, selection may be made as follows. Namely, the library DNA is used to transform Escherichia coli JM109 (produced by Takara Shuzo). Obtained transformants are used to carry out a method of colony hybridization [R. Bruce Wallace et al., Nuc. Acid Res., Vol. 9, p. 879 (1981)] by using the probe prepared in the foregoing item (1).

Phage DNA or plasmid DNA is extracted from the transductant or the transformant selected as described above, and an inserted fragment is excised from the vector by using an appropriate restriction enzyme. Thus a DNA fragment containing DNA of the present invention can be obtained.

Table 2 shows the size of DNA fragments originating from respective microbial strains obtained in Examples described later on in accordance with the aforementioned procedure, and restriction enzymes used for digestion.

**Table 2**

| fragment | Restriction enzyme | Size of DNA |
|---|---|---|
| Alcaligenes faecalis IFO 13111 | XhoI | about 2 kb |
| Bacillus stearothermophilus MI-101 | EcoRI | about 4 kb |
| Bacillus stearothermophilus MI-102 | HindIII | about 2 kb |
| Bacillus brevis MI-103 | FabI | about 4.5 kb |
| Bacillus stearothermophilus MI-105 | EcoRI | about 1.9 kb |
| Bacillus stearothermophilus MI-110 | PstI | about 2.9 kb |
| Serratia marcescens IFO 3736 | PstI | about 1.9 kb |

The existence of the maleate isomerase gene in the inserted DNA fragment can be confirmed again for the DNA fragment excised by the operation described above by performing Southern hybridization [E. M. Southern, J. Mol. Biol., Vol. 98, p. 503 (1975)] by using the probe prepared in the foregoing item (1), for example, the probe having the nucleotide sequence shown in Table 1.

### (3) Determination of nucleotide sequence:

An entire nucleotide sequence of the DNA fragment obtained in the foregoing item (2) can be determined in accordance with a dideoxy chain termination method [Sanger, F. et al., Proc. Natl. Acad. Sci. USA, Vol. 74, p. 5463 (1977)] by using, for example, a plasmid vector such as pBluescript (produced by Stratagene) and pUC118 (produced by Takara Shuzo).

The existence of an open reading frame (ORF) is confirmed in the nucleotide sequence of the DNA fragment thus determined as described above, and it has been revealed that the maleate isomerase genes of the respective strains have structures as shown in Table 3. It has been confirmed again that the obtained nucleotide sequences (SEQ ID NOs: 4, 11, 18, 25, 32, 39, 46) contain sequences corresponding to nucleotide sequences deduced from the amino acid sequences represented by the sequences shown in Table 1 (SEQ ID NOs: 6, 13, 20, 27, 34, 41, 48).

**Table 3**

| Source | SEQ ID NO: | Number of encoded amino acids | Length of ORF (base pairs) |
|---|---|---|---|
| Alcaligenes faecalis IFO 13111 | 4 | 253 | 759 |
| Bacillus stearothermophilus MI-101 | 11 | 251 | 753 |
| Bacillus stearothermophilus MI-102 | 18 | 251 | 753 |
| Bacillus brevis MI-103 | 25 | 252 | 756 |
| Bacillus stearothermophilus MI-105 | 32 | 251 | 753 |
| Bacillus stearothermophilus MI-110 | 39 | 251 | 753 |
| Serratia marcescens IFO 3736 | 46 | 250 | 750 |

The maleate isomerase gene of the present invention including the nucleotide sequences described above is not limited to only those separated from chromosomal DNA of natural bacteria. It may be those synthesized by using an ordinarily used DNA synthesizer, for example, 394 DNA/RNA synthesizer produced by Applied Biosystems.

### (4) Construction of recombinant vector having maleate isomerase gene, and transformant harboring the same recombinant vector:

The maleate isomerase gene of the present invention or the DNA fragment containing the maleate isomerase gene can be expressed by ligating the gene or the fragment with an appropriate vector to prepare a recombinant vector, and transforming an appropriate host microorganism with the recombinant vector. Usually, when the recombinant vector is prepared, the maleate isomerase gene is inserted into a vector together with a promoter suitable for a host microorganism so that a 5'-terminal side of a coding region of the gene is ligated at a position downstream from the promoter. Alternatively, an expression vector containing a promoter may be used, into which the maleate isomerase gene may be inserted.

Other than the expression method to use the expression vector as described above, the gene of the present invention can be also expressed in accordance with a homologous recombination technique in which a DNA fragment containing the maleate isomerase gene ligated with a promoter is directly introduced into chromosome of a host microorganism [for example, A. A. Vertes et al., Biosci. Biotechnol. Biochem., Vol. 57, p. 2036 (1993)], or an introduction technique by using a transposon or an inserted sequence [for example, A. A. Vertes et al., Molecular Microbiol., Vol. 11, p. 739 (1994)].

The expression vector is not specifically limited provided that it is reproducible and proliferative in the host microorganism. Any one of a plasmid vector and a phage vector may be used. Specifically, the plasmid vector includes, for example, pBR322, pUC18, pHSG298, pUC118, pSTV28, pTWV228, pHY300PLK (the foregoing plasmid vectors are commercially available from, for example, Takara Shuzo), pKK223-3, pPL-Lambda Inducible Expression Vector (the foregoing plasmid vectors are commercially available from, for example, Pharmacia), pCRY31, pCRY3KE, pCRY3KX (United States Patent No. 5,185,262), and derivatives thereof.

The phage vector includes, for example, λFixII vector (commercially available from Stratagene).

A promoter harbored by a host microorganism can be generally used as the promoter for expressing the maleate isomerase gene of the present invention. However, there is no limitation thereto. Any promoter may be used provided that it resides in a nucleotide sequence originating from a prokaryote for starting transcription of the maleate isomerase gene. Specifically, the promoter includes, for example, a promoter of lactose operon, a promoter of tryptophan operon, PL promoter originating from λ phage, and a tryptophan-lactose hybrid (tac) promoter [H. A. Bose et al., Proc. Natl. Acad. Sci. U.S.A., Vol. 80, p. 21 (1983)]. Alternatively, when a microorganism, in which an inherent promoter of the obtained maleate isomerase gene is operable, is used as the host, the promoter may be used as it is. Further alternatively, it is also allowable to use those synthesized by using an ordinarily used DNA synthesizer, for example, 394 DNA/RNA synthesizer produced by Applied Biosystems.

Among the foregoing promoters, an inducible promoter may be used to increase the expression efficiency by using an inducing factor thereof (for example, a substance, or a growth environment such as temperature). In the case of the use of the promoter of lactose operon described above, for example, the gene expression can be induced by adding lactose or isopropyl-β-D-thiogalactoside (IPTG).

The host into which the maleate isomerase gene is introduced is not specifically limited. However, those preferably used include, for example, bacteria belonging to the genera Escherichia, Bacillus, Serratia, Pseudomonsa, Corynebacterium, Brevibacterium, Rhodococcus, Lactobacillus, Streptomyces, Thermus, and Streptococcus.

Specifically, those usable as the host include, for example, Escherichia coli, Bacillus subtilis, Bacillus brevis, Bacillus stearothermophilus, Serratia marcescens, Pseudomonas putida, Pseudomonas aeruqinosa, Corynebacterium glutamicum, Brevibacterium flavum, Brevibacterium lactofermentum, Rhodococcus erythropolis, Thermus thermophilus, Streptococcus lactis, Lactobacillus casei, and Streptomyces lividans.

Those usable as a method for introducing the gene into the host microorganism described above include, for example, transformation methods based on, for example, competent cell methods [Journal of Molecular Biology, Vol. 53, p. 159 (1970)] and electroporation methods [J. Indust. Microbiol., Vol. 5, p. 159 (1990)], transduction methods based on the use of phage [E. Ohtsubo, Genetics, Vol. 64, p. 189 (1970)], conjugative transfer methods [J. G. C. Ottow, Ann. Rev. Microbiol., Vol. 29, p. 80 (1975)], and cell fusion methods [M. H. Gabor, J. Bacteriol., Vol. 137, p. 1346 (1979)].

### (5) Production of maleate isomerase by using transformant of the present invention, and isomerization reaction from maleic acid to fumaric acid by using maleate isomerase:

Maleic acid can be isomerized to produce fumaric acid by adding, to an aqueous solution containing maleic acid, the transformant obtained as described in the foregoing item (4) or a crude enzyme fraction or a purified enzyme of maleate isomerase collected from a culture of the transformant. The culture herein refers to microbial cells of the transformant and/or a medium after cultivation (culture supernatant when a liquid medium is used for cultivation).

The transformant usable for the method for producing fumaric acid according to the present invention includes microbial cells separated from a culture liquid obtained by cultivating the transformant as a matter of course, as well as a culture liquid, a disrupted preparation of microbial cells, and an extract from microbial cells. A crude enzyme sample obtained from the transformant may be used, or a purified enzyme sample may be used. Further, it is possible to use those obtained by immobilizing, on a carrier, the transformant described above, a disrupted preparation or an extract thereof, or a purified enzyme.

The transformant described above can be cultivated by using an ordinary nutrient medium containing, for example, a carbon source, a nitrogen source, inorganic salts, and various vitamins. Those usable as the carbon source include, for example, sugar such as glucose, sucrose, fructose, and maltose; alcohol such ethanol and methanol; organic acid such as citric acid, malic acid, succinic acid, maleic acid, and fumaric acid; and waste molasses. Those usable as the nitrogen source singly or in mixture include, for example, ammonia, ammonium sulfate, ammonium chloride, ammonium nitrate, and urea. Those usable as the inorganic salts include, for example, dipotassium hydrogenphosphate, potassium dihydrogenphosphate, and magnesium sulfate. Other than the above, it is possible to add, to the medium, nutrients such as peptone, meat extract, yeast extract, corn steep liquor, casamino acid, and various vitamins such as biotin.

The cultivation is usually performed under an aerobic condition achieved by, for example, aeration, agitation, and shaking. The cultivation temperature is not specifically limited provided that the host microorganism is growable at that temperature. The pH during the cultivation is also not specifically limited provided that the host microorganism is growable at that pH. The pH can be adjusted during the cultivation by adding acid or alkali.

Microbial cells which contain the expression product of the maleate isomerase gene, i.e., maleate isomerase can be obtained by collecting the microbial cells from a culture obtained as described above by means of, for example, centrifugation.

When the transformant is immobilized on a carrier, microbial cells recovered from a culture may be used as they are, or microbial cells washed with an appropriate buffer, for example, phosphate buffer of about 0.02 to 0.2 M (pH 6 to 10) may be used. Those usable for the production of fumaric acid according to the present invention include, for example, those obtained by immobilizing, on a carrier, a disrupted preparation obtained by disrupting microbial cells recovered from a culture by means of, for example, ultrasonication or pressurized squeezing or milling, an extract containing maleate isomerase obtained by extracting the disrupted preparation with, for example, water, and a partially purified fraction of maleate isomerase obtained from the extract by means of treatments such as ammonium sulfate salting-out and column chromatography as well.

The microbial cells, the disrupted preparation of microbial cells, the extract, or the purified preparation can be immobilized in accordance with a per se known and usually used method, based on a method for immobilizing microbial cells or the like on an appropriate carrier such as acrylamide monomer, alginic acid, and carageenan.

The aqueous solution to be used for the reaction may be an aqueous solution or an appropriate buffer containing maleic acid, such as phosphate buffer of about 0.02 to 0.2 M (pH 6 to 10). In order to increase the substance permeability of cell membranes of the microbial cells, the aqueous solution may be further added with, for example, toluene, xylene, and nonionic surfactant in an amount of 0.05 to 2 % (w/v).

Maleic acid, which serves as a raw material for the reaction in the aqueous solution, is appropriately at a concentration of about 0.1 to 2 M. The usable form of maleic acid include a form of acid or salt, as well as anhydride thereof. Maleic anhydride is readily converted into maleic acid by preparing an aqueous solution thereof. Salts of maleic acid include, for example, sodium salt and potassium salt.

The temperature and the pH for the enzyme reaction in the aqueous solution described above are not specifically limited. However, the temperature may be usually 10 to 60 °C, appropriately 20 to 50 °C, and the pH of the reaction solution may be 5 to 10, preferably about 6 to 9. The pH can be adjusted by adding acid or alkali.

An aqueous solution containing fumaric acid thus obtained may be used as it is as a raw material for producing, for example, malic acid and aspartic acid.

The present invention has been explained in the foregoing description. The present invention will be more specifically explained with reference to Examples described below. However, Examples should be regarded as the aid to obtain concrete recognition of the present invention, which do not limit the scope of the present invention at all.

### Brief Description of the Drawings

Fig. 1 represents the amino acid sequence of maleate isomerase of the present invention in a unified manner.

Fig. 2 illustrates comparison among amino acid sequences of maleate isomerases originating from various microorganisms.

### Best Mode for Carrying Out the Invention

### Example 1: Separation of Microorganisms Having Maleate Isomerase Activities

### (1) Separation of maleic acid-assimilating microorganisms

Soil samples collected from the nature were introduced into a medium A [10 ml, composition: disodium maleate 5 g, KH₂PO₄ 0.7 g, K₂HPO₄ 1.4 g, NH₄NO₃ 1 g, MgSO₄·7H₂O 0.2 g, biotin 0.2 mg, thiamin hydrochloride 0.2 mg, FeSO₄·7H₂O 20 mg, MnSO₄ 20 mg, distilled water 1 L (pH 7.2)] respectively, and aerobically cultivated with shaking at 50 °C for 3 days. Obtained cultures (1 ml) were subcultured to the medium A (10 ml) respectively, and they were cultivated in the same manner for further 3 days. Cultures obtained after the cultivation were spread over a plate medium having the composition of the medium A containing 2 % agar respectively, and they were cultivated at 50 °C for 2 days. Colonies grown on the plate medium were isolated.

### (2) Selection of microorganisms having maleate isomerase activities

Microorganisms isolated in the foregoing item (1) were cultivated respectively at 50 °C for 1 day by using 100 ml of the medium A. Microbial cells were recovered by centrifugation (3,000 x g, 4 °C, 20 minutes), and then they were washed with 0.9 % (w/v) NaCl. Next, the washed microbial cells were suspended in 1 ml of the same NaCl solution, to which a reaction solution [1 ml, composition: sodium maleate 84 g/l, Triton X-100 1 g/l] was added, followed by shaking overnight at 50 °C to recover supernatant solutions by centrifugation. The obtained supernatants of the reaction solutions were subjected to high-performance liquid chromatography analysis (LC-5A, produced by Shimadzu) by using a column for organic acid analysis (SCR-101H column produced by Shimadzu) and a UV detector (210 nm). Some microorganisms having enzyme activities of maleate isomerase were selected from the respective microorganisms isolated in the foregoing item (1). As for the selected microorganisms, a peak of maleic acid as the raw material was decreased, and a peak of fumaric acid was generated.

Microbiological properties were investigated for the respective microorganisms having the maleate isomerase activity obtained by the aforementioned separation operation in accordance with Bergey's Manual of Systematic Bacteriology. As a result, they were revealed to be novel microbial strains, and designated as Bacillus stearothermophilus MI-101, MI-102, MI-105, MI-110, and Bacillus brevis MI-103 respectively. The strains of MI-101, MI-102, MI-103, and MI-105 have been deposited on March 7, 1995, and the strain of MI-110 has been deposited on November 28, 1995 in National Institute of Bioscience and Human Technology of Agency of Industrial Science and Technology of Ministry of International Trade and Industry (postal code: 305, 1-3, Higashi 1-chome, Tsukuba-shi, Ibaraki-ken, Japan) under deposition numbers of FERM P-14801, FERM P-14802, FERM P-14803, FERM P-14805, and FERM P-15313 respectively in this order. The strains of MI-101, MI-102, MI-103, and MI-105 were transferred on July 12, 1995, and the strain of MI-110 was been transferred on December 15, 1995 to international deposition based on Budapest Treaty. The transferred strains have been deposited under deposition numbers of FERM BP-5160, FERM BP-5161, FERM BP-5162, FERM BP-5164, and FERM BP-5339 respectively in this order.
(a) Bacillus stearothermophilus MI-101
   1. Morphological properties:
      shape: rod;
      spore: formed (oval, position: terminal to subterminal, sporangium: not distend);
      Gram stain: positive;
      motility: absent.
   2. Physiological properties:
      attitude to oxygen: aerobic;
      growth under anaerobic condition: negative;
      catalase: positive;
      V-P reaction: negative;
      pH of V-P broth: 5.6;
      production of acid from glucose: positive;
      production of gas from glucose: negative;
      liquefaction of gelatin: positive;
      decomposition of starch: positive;
      utilization of citrate: positive;
      utilization of propionate: negative;
      egg yolk reaction: negative;
      reduction of nitrate: positive;
      growth at pH 6.8 (nutrient broth): positive;
      growth at pH 5.7: negative;
      growth in the presence of 5 % NaCl: negative;
      growth in the presence of 7 % NaCl: negative;
      growth at 10 °C: negative;
      growth at 30 °C: negative;
      growth at 40 °C: positive;
      growth at 55 °C: positive,
      growth at 65 °C: positive;
      growth at 70 °C: positive;
      maleate isomerase activity: present;
      GC content of intracellular DNA (mole %): 42 %.
(b) Bacillus stearothermophilus MI-102
   1. Morphological properties:
      shape: rod;
      spore: formed (oval, position: terminal to subterminal, sporangium: not distend);
      Gram stain: positive;
      motility: present.
   2. Physiological properties:
      attitude to oxygen: aerobic;
      growth under anaerobic condition: negative;
      catalase: positive;
      V-P reaction: extremely weak;
      pH of V-P broth: 5.5;
      production of acid from glucose: positive;
      production of gas from glucose: negative;
      liquefaction of gelatin: positive;
      decomposition of starch: positive;
      utilization of citrate: positive;
      utilization of propionate: negative;
      egg yolk reaction: negative;
      reduction of nitrate: positive;
      growth at pH 6.8 (nutrient broth): positive;
      growth at pH 5.7: positive;
      growth in the presence of 5 % NaCl: negative;
      growth in the presence of 7 % NaCl: negative;
      growth at 10 °C: negative;
      growth at 30 °C: negative;
      growth at 40 °C: positive;
      growth at 55 °C: positive;
      growth at 65 °C: positive;
      growth at 70 °C: positive;
      maleate isomerase activity: present;
      GC content of intracellular DNA (mole %): 54 %.
(c) Bacillus brevis MI-103
   1. Morphological properties:
      shape: rod;
      spore: formed (oval, position: terminal to subterminal, sporangium: distends a little);
      Gram stain: positive;
      motility: present.
   2. Physiological properties:
      attitude to oxygen: aerobic;
      growth under anaerobic condition: negative;
      catalase: positive;
      V-P reaction: negative;
      pH of V-P broth: 7.2;
      production of acid from glucose: positive;
      production of gas from glucose: negative;
      liquefaction of gelatin: negative;
      decomposition of starch: negative;
      utilization of citrate: positive;
      utilization of propionate: positive;
      egg yolk reaction: negative;
      reduction of nitrate: positive;
      growth at pH 6.8 (nutrient broth): positive;
      growth at pH 5.7: negative;
      growth in the presence of 5 % NaCl: negative;
      growth in the presence of 7 % NaCl: negative;
      growth at 30 °C: positive;
      growth at 40 °C: positive;
      growth at 55 °C: positive;
      growth at 65 °C: negative;
      growth at 70 °C: negative;
      maleate isomerase activity: present;
      GC content of intracellular DNA (mole %): 51 %.
(d) Bacillus stearothermophilus MI-105
   1. Morphological properties:
      shape: rod;
      spore: formed (oval, position: terminal to subterminal, sporangium: distends a little);
      Gram stain: positive;
      motility: present.
   2. Physiological properties:
      attitude to oxygen: aerobic;
      growth under anaerobic condition: negative;
      catalase: positive;
      V-P reaction: negative;
      pH of V-P broth: 6.1;
      production of acid from glucose: positive;
      production of gas from glucose: negative;
      liquefaction of gelatin: positive;
      decomposition of starch: positive;
      utilization of citrate: negative;
      utilization of propionate: negative;
      egg yolk reaction: negative;
      reduction of nitrate: negative;
      growth at pH 6.8 (nutrient broth): positive;
      growth at pH 5.7: negative;
      growth in the presence of 5 % NaCl: negative;
      growth in the presence of 7 % NaCl: negative;
      growth at 10 °C: negative;
      growth at 30 °C: negative;
      growth at 40 °C: positive;
      growth at 55 °C: positive;
      growth at 65 °C: positive;
      maleate isomerase activity: present;
      GC content of intracellular DNA (mole %): 40 %.
(e) Bacillus stearothermophilus MI-110
   1. Morphological properties:
      shape: rod;
      spore: formed (oval, position: terminal to subterminal, sporangium: distends a little);
      Gram stain: positive;
      motility: present.
   2. Physiological properties:
      attitude to oxygen: aerobic;
      growth under anaerobic condition: negative;
      catalase: positive;
      V-P reaction: negative;
      pH of V-P broth: 5.7;
      production of acid from glucose: positive;
      production of gas from glucose: negative;
      liquefaction of gelatin: positive;
      decomposition of starch: positive;
      utilization of citrate: positive;
      utilization of propionate: positive;
      egg yolk reaction: negative;
      reduction of nitrate: positive;
      growth at pH 6.8 (nutrient broth): positive;
      growth at pH 5.7: negative;
      growth in the presence of 5 % NaCl: negative;
      growth in the presence of 7 % NaCl: negative;
      growth at 30 °C: negative;
      growth at 40 °C: positive;
      growth at 65 °C: positive;
      maleate isomerase activity: present;
      GC content of intracellular DNA (mole %): 53 %.

### (2) Properties of maleate isomerase produced by respective selected microbial strains belonging to the genus Bacillus

### (i) Purification of maleate isomerase

The medium A [3 L, composition: disodium maleate 5 g, KH₂PO₄ 0.7 g, K₂HPO₄ 1.4 g, NH₄NO₃ 1 g, MgSO₄·7H₂O 0.2 g, biotin 0.2 mg, thiamin hydrochloride 0.2 mg, FeSO₄·7H₂O 20 mg, MnSO₄ 20 mg, distilled water 1 L (pH 7.2)] was introduced into a jar fermenter having a volume of 5 L, and the medium was subjected to a sterilization treatment at 121 °C for 20 minutes, to which the respective microbial strains were seeded and cultivated at 50 °C for 24 hours with shaking. After the cultivation, microbial cells were recovered from culture liquids by centrifugation (3,000 x g, 4 °C, 20 minutes) respectively, and they were washed with a buffer A [composition: 200 mM potassium phosphate buffer (pH 7.2), 0.5 mM dithiothreitol (threo-1,4-dimercapto-2,3-butanediol)].

The obtained microbial cells were suspended in the buffer A again to obtain a microbial cell suspension of about 30 % (w/v) which was treated with an ultrasonic disrupter (produced by Branson) to disrupt the microbial cells. Next, an obtained disrupted preparation of the microbial cells was centrifuged (12,000 x g, 4 °C, 30 minutes) to recover a supernatant as a crude enzyme solution containing maleate isomerase.

Streptomycin sulfate was added to the obtained crude enzyme solution to give a concentration of 2.2 % (w/v) in order to remove nucleic acid. After that, a fraction of 30 to 70 % ammonium sulfate precipitation was recovered in accordance with an ordinary method, which was dialyzed overnight in the buffer A (4 °C). The obtained ammonium sulfate fraction was adsorbed to a DEAE-Sephacel (produced by Pharmacia) column (diameter: 2.6 cm x 30 cm) equilibrated with the buffer A, which was sufficiently washed with the buffer A, and then eluted with a linear concentration gradient (0 to 0.2 M) of potassium chloride. The flow rate was 2 ml/minute, and respective fractions were collected in an mount of 10 ml for each. Fractions containing maleate isomerase were detected by measuring the maleate isomerase activity on the basis of the decrease in substrate (maleic acid) as the decrease in absorbance at 240 nm in the presence of swine heart fumarase (produced by Boehringer Mannheim) by using a spectrophotometer (DU 7500 produced by Beckman) in accordance with a method of Otsuka et al. [Agric. Biol. Chem., Vol. 25, p. 726 (1961)].

An obtained active fraction was concentrated by using an ultrafiltration apparatus (UHP-43K produced by Toyo Filter Paper, fractionating molecular weight: 20,000), and then charged to a gel filtration column (Sephacryl S-200 produced by Pharmacia, ⌀ 1.6 x 60 cm) equilibrated with the buffer A, followed by elution at a flow rate of 0.5 ml. Active fractions were detected in the same manner as described above. Next, an obtained active fraction was charged to an anion exchange column (Mono-Q produced by Pharmacia, ⌀ 1 x 10 cm) equilibrated with the buffer A, and eluted with a linear concentration gradient (0 to 1 M) of potassium chloride. An obtained active fraction was used as a purified fraction of maleate isomerase.

The purified fraction of maleate isomerase was subjected to polyacrylamide gel electrophoresis in accordance with the method of Davis and the method of Laemmli described above, and stained with a Coomassie Blue dyestuff solution [composition: 0.2 % (w/v) Coomassie Brilliant Blue R250, 40 % (v/v) methanol, 10 % (v/v) acetic acid]. As a result, an approximately single band was detected in any of the methods described above.

### (ii) Measurement of maleate isomerase activity

A reaction was started by adding, to the buffer A (1,850 µl), a solution (50 µl) of the purified maleate isomerase obtained in the foregoing item (i), keeping the temperature at 50 °C, and adding and mixing a solution (100 µl) of 200 mM disodium maleate. The reaction velocity was determined as a velocity to produce fumaric acid by measuring the velocity of increase in absorbance at 290 nm for 5 minutes by using a spectrophotometer (DU-7500 produced by Beckman). The reaction velocity was also measured at 40 °C and 60 °C in the same manner as described above. The maleate isomerase activity at each temperature was calculated as a relative activity obtained by regarding a reaction velocity at 50 °C as 100. Results are shown in Table 4.

**Table 4**

| | Relative activity at respective temperatures | | |
|---|---|---|---|
| | 40 °C | 50 °C | 60 °C |
| Bacillus stearothermophilus MI-101 | 67 | 100 | 130 |
| Bacillus stearothermophilus MI-102 | 80 | 100 | 120 |
| Bacillus brevis MI-103 | 72 | 100 | 110 |
| Bacillus stearothermophilus MI-105 | 65 | 100 | 113 |
| Bacillus stearothermophilus MI-110 | 80 | 100 | 120 |

### Example 2: Isolation of Maleate Isomerase Gene Originating from Alcaligenes faecalis IFO 13111 Strain and Construction of Transformant

### (1) Cloning of DNA fragment containing maleate isomerase gene:

### (1)-1 Purification of maleate isomerase, determination of partial amino acid sequence, and preparation of DNA probe based thereon:

A maleic acid-bouillon medium [100 ml, composition: sodium maleate 10 g, beef extract 10 g, NaCl 5 g, distilled water 1,000 ml (adjusted at pH 7)] was dispensed and poured into an Erlenmeyer flask having a volume of 500 ml, and the medium was subjected to a sterilization treatment at 120 °C for 20 minutes, to which Alcaligenes faecalis IFO 13111 strain was seeded, followed by cultivation at 30 °C for 24 hours with shaking.

Next, the same medium as described above (1,000 ml) was introduced into a jar fermenter having a volume of 3 L, and the medium was subjected to a sterilization treatment at 120 °C for 20 minutes, to which a culture obtained as described above (30 ml) was seeded, followed by cultivation at 30 °C for 24 hours with aeration and agitation. A culture liquid after the cultivation was centrifuged (3,500 x g, 4 °C, 20 minutes) to recover microbial cells.

Maleate isomerase was extracted from the obtained microbial cells by suspending the microbial cells in a phosphate buffer (20 mM, pH 7.2) containing 0.5 mM dithiothreitol, and disrupting the microbial cells by using an ultrasonic disrupter (produced by Branson). A preparation of the disrupted microbial cells was centrifuged (10,000 x g, 4 °C, 30 minutes) to obtain a soluble fraction of a supernatant as a crude enzyme solution.

Next, the crude enzyme solution was subjected to ammonium sulfate fractionation (40 to 70 %), and an obtained fraction was dialyzed against the aforementioned phosphate buffer at 4 °C overnight.

Next, this dialyzed preparation was subjected to DEAE-Sepharose (produced by Pharmacia) column chromatography, gel filtration (Sephacryl S-200, produced by Pharmacia) column chromatography, and Mono-Q (produced by Pharmacia) column chromatography to obtain an active fraction of maleate isomerase.

The activity of maleate isomerase was detected by measuring the decrease in maleic acid as the change in absorbance at 240 nm in the presence of swine heart fumarase (produced by Boehringer Mannheim) in accordance with a method of Otsuka et al. [Agric. Biol. Chem., Vol. 25, p. 726 (1961)].

The purified fraction of maleate isomerase described above was separated by polyacrylamide gel (4 to 20 % gradient gel) electrophoresis (electrophoresed at a constant current of 40 mA for 1 hour in a buffer (pH 8.4) comprising 25 mM Tris-192 mM glycine), followed by staining with Coomassie Blue dyestuff [composition: 0.2 % (w/v) Coomassie Brilliant Blue R250, 40 % (v/v) methanol, 10 % (v/v) acetic acid]. As a result, a single band having a molecular weight of about 60 kDa was detected. Further, the purified fraction was dissolved in a treatment solution of sodium dodecyl sulfate (SDS) [composition: 62.5 mM Tris-HCl (pH 6.8), 2 % SDS, 10 % glycerol, 5 % 2-mercaptoethanol, 0.001 % Bromophenol Blue (BPB)], and subjected to a heat denaturation treatment at 95 °C for 3 minutes, followed by separation by polyacrylamide gel (10 to 20 % gradient gel) electrophoresis (electrophoresed at a constant current of 40 mA for 1 hour in a buffer (pH 8.4) comprising 25 mM Tris-192 mM glycine-0.1 % SDS) to perform staining in the same manner as described above. As a result, only a band having a molecular weight of about 30 kDa was detected. According to the foregoing results, it is assumed that the structure of maleate isomerase is a dimer having a molecular weight of about 60 kDa comprising subunits having a molecular weight of about 30 kDa.

An amino acid sequence from the N-terminal of the purified isomerase was determined by using an amino acid sequencer (473A type, produced by Applied Biosystems). A result is shown in SEQ ID NO: 6.

Next, probe DNA's having nucleotide sequences shown in SEQ ID NOs: 7 and 8 deduced from the obtained amino acid sequence were synthesized by using a DNA synthesizer (394 type, produced by Applied Biosystems).

### (1)-2 Preparation of chromosomal DNA fragment containing maleate isomerase gene:

Alcaligenes faecalis IFO 13111 strain was cultivated with shaking at 30 °C by using a maleic acid-bouillon medium [composition: sodium maleate 10 g, beef extract 10 g, NaCl 5 g, distilled water 1,000 ml (adjusted at pH 7)] until a late logarithmic growth phase to collect microbial cells.

The obtained microbial cells were suspended in 15 ml of a solution of 10 mM NaCl-20 mM Tris buffer (pH 8.0)-1 mM EDTA·2Na containing lysozyme so that the concentration was 10 mg/ml. Next, proteinase K (produced by Takara Shuzo) was added so that its final concentration was 100 µg/ml, and the temperature was kept at 37 °C for 1 hour. Further, sodium dodecyl sulfate (SDS) was added so that its final concentration was 0.5 %, and the temperature was kept at 50 °C for 6 hours to achieve bacteriolysis. An equal amount of a phenol/chloroform solution was added to an obtained lysate to perform gentle shaking at room temperature for 10 minutes. After that, the entire amount was centrifuged (5,000 x g, 20 minutes, 10 to 12 °C) to fractionate a supernatant fraction. Sodium acetate was added to the supernatant so that its concentration was 0.3 M, to which a 2-fold amount of ethanol was then slowly added. DNA existing between aqueous and ethanol layers was wound and collected by using a glass rod, which was washed with 70 % ethanol, followed by drying in air. A solution (5 ml) of 10 mM Tris buffer (pH 7.5)-1 mM EDTA·2Na was added to the obtained DNA, followed by being left to stand at 4 °C overnight to obtain a chromosomal DNA solution.

Next, a restriction enzyme XhoI (50 U (units)) was added to 90 µl of the chromosomal DNA solution to make a reaction at 37 °C for 1 hour so that complete degradation was achieved. An obtained solution was subjected to agarose gel electrophoresis. DNA was transferred from the agarose gel to a nylon membrane to perform Southern hybridization in accordance with an ordinary method ["Molecular Cloning", Cold Spring Harbor Laboratory Press (1989)] by using the synthetic probes shown in SEQ ID NOs: 7 and 8 with their 5'-terminal phosphate groups radioisotope-labeled [Anal. Biochem., 158, 307-315 (1986)] by using T4 polynucleotide kinase (produced by Takara Shuzo) and [γ-³²P]ATP.

As a result, it was confirmed that a DNA fragment, to which the synthetic probes strongly hybridized, was present at a position of about 2 kb on the nylon membrane.

### (1)-3 Insertion of chromosomal DNA into vector:

A restriction enzyme XhoI (5 units) was added to the chromosomal DNA solution (90 µl) of Alcaligenes faecalis IFO 13111 strain obtained in the foregoing item (1)-2 to perform partial degradation by making a reaction at 37 °C for 10 minutes. Partially degraded DNA's having various lengths were mixed with a phage vector λFIXII (produced by Toyobo) cleaved with the restriction enzyme XhoI, to which respective components of 50 mM Tris buffer (pH 7.6), 10 mM dithiothreitol, 1 mM ATP, 10 mM MgCl₂, and T4 DNA ligase (1 unit) were added (concentrations of the respective components were final concentrations) to make a reaction at 16 °C for 10 hours. Thus the partially degraded DNA's were ligated with the vector to obtain a λDNA library of the chromosomal DNA.

### (1)-4 Selection of objective recombinant DNA:

A phage solution of the λDNA library prepared in the foregoing item (1)-3 (2-5 x 10⁴ pfu; diluted with SM buffer) was mixed with an equivalent amount of a culture of Escherichia coli P2392, which was kept at a temperature of 37 °C for 15 minutes. A λ medium (3 to 4 ml, 1 % Trypton, 0.5 % yeast extract, 0.5 % NaCl, 0.2 % MgSO₄·7H₂O, 0.5 % agar) having been kept at a temperature of 50 °C was added to the mixed solution, which was homogeneously applied to a λ plate (1 % Trypton, 0.5 % NaCl, 0.2 % MgSO₄·7H₂O, 1 % agar), followed by cultivation at 37 °C for 12 to 16 hours.

A nitrocellulose filter was placed on the medium so that plaques formed on the medium were adsorbed to the filter. The filter was treated by successively placing it for every 5 minutes on sheets of filter paper immersed in the following reagents (a) to (c).
(a) 0.5 M NaOH, 1.5 M NaCl;
(b) 0.5 M Tris-HCl (pH 7.5), 1.5 M NaCl, 0.001 M EDTA;
(c) 2 x SSC (20 x SSC: NaCl 175.3 g, trisodium citrate dihydrate 88.2 g dissolved in 1 L of distilled water).

The filter was dried in air, and then it was treated in hot air in a dry state at 80 °C for 2 hours to immobilize DNA.

The filter prepared as described above was subjected to plaque hybridization by using the probes shown in SEQ ID NOs: 7 and 8 prepared in the foregoing item (1)-1 in accordance with an ordinary method ["Molecular Cloning", Cold Spring Harbor Laboratory Press (1989)].

As a result, a hybridization-positive plaque for the both probes was selected, from which phage DNA was extracted to confirm an inserted fragment having a length of about 2 kb excised by the restriction enzyme XhoI by means of agarose gel electrophoresis.

In order to further specify the position of the maleate isomerase gene existing on the obtained DNA (XhoI-XhoI) fragment having the length of about 2 kb, the DNA fragment was subcloned into a plasmid pBluescript IIKS+ (produced by Toyobo) as follows.

The DNA fragment excised with XhoI and the cloning vector pBluescript KS+ (produced by Toyobo) were digested with the restriction enzyme XhoI, and then subjected to a dephosphorylation treatment respectively. The both were mixed, to which respective components of 50 mM Tris buffer (pH 7.6), 10 mM dithiothreitol, 1 mM ATP, 10 mM MgCl₂, and T4 DNA ligase (1 unit) were added (concentrations of the respective components were final concentrations) to make a reaction at 16 °C for 10 hours so that the XhoI-XhoI fragment was ligated with the vector.

Escherichia coli JM109 (produced by Takara Shuzo) was transformed with a solution obtained by the ligation reaction in accordance with a calcium chloride method [Journal of Molecular Biology, Vol. 53, p. 159 (1970)], which was spread over a medium [Trypton 10 g, yeast extract 5 g, NaCl 5 g, and agar 16 g dissolved in 1 L of distilled water] containing 50 µg/ml of ampicillin.

A strain grown on the medium was cultivated in a liquid medium in accordance with an ordinary method, and plasmid DNA was extracted from an obtained culture. The plasmid was digested with the restriction enzyme XhoI to investigate an inserted fragment by using a hybridization method. As a result, a DNA fragment having a length of about 2 kb was confirmed in addition to a DNA fragment having a length of 3 kb of the plasmid pBluescript IIKS+.

The transformant obtained as described above was seeded to the LB medium (liquid medium from which agar was removed) containing 50 µg/ml of ampicillin, 5 g/L of sodium maleate, and 1 g/L of malonic acid, and cultivated aerobically with shaking at 37 °C for 15 hours. An obtained culture was centrifuged (3,000 x g, 4 °C, 20 minutes) to recover microbial cells which were then washed with a phosphate buffer [composition: 20 mM potassium phosphate buffer (pH 7.2), 0.5 mM dithiothreitol].

Next, the washed microbial cells (0.5 g, wet weight) were suspended in the phosphate buffer (2 ml), which were treated with an ultrasonic disrupter (produced by Branson) under cooling with ice to obtain a disrupted preparation of the microbial cells. The disrupted preparation was centrifuged (10,000 x g, 4 °C, 30 minutes) to obtain a supernatant as a crude enzyme solution. The crude enzyme solution was used to measure the decrease in maleic acid as the change in absorbance at 240 nm in the presence of swine heart fumarase (produced by Boehringer Mannheim) in the same manner as described in the foregoing item (1) [K. Otsuka, Agric. Biol. Chem., 25, 726 (1961)]. As a result, we succeeded in detecting the maleate isomerase activity.

### (2) Determination of nucleotide sequence:

A nucleotide sequence of the DNA (XhoI-XhoI) fragment having the length of about 2 kb containing the maleate isomerase gene obtained in the foregoing item (1)-4 was determined in accordance with a dideoxychain termination method [Sanger, F. et al., Proc. Natl. Acad. Sci. USA, Vol. 74, p. 5463 (1977)] by using pBluescript IIKS+ (produced by Toyobo).

According to the existence of an open reading frame in the determined nucleotide sequence, it has been revealed that the maleate isomerase gene has a nucleotide sequence shown in SEQ ID NO: 4 in Sequence Listing described later on and comprises 759 base pairs coding for 253 amino acids. The amino acid sequence is shown in SEQ ID NO: 5.

It has been confirmed that the determined nucleotide sequence contains a sequence corresponding to the entire nucleotide sequence deduced from the amino acid sequence (SEQ ID NO: 6) determined in the item (1)-1.

### (3) Preparation of expression plasmid and construction of transformant:

A gene region (about 800 bp) coding for maleate isomerase was amplified by means of a PCR (Polymerase Chain Reaction) method on the basis of the nucleotide sequence of SEQ ID NO: 4 determined in the item (2), which was inserted into an expression vector autonomously replicable in Escherichia coli as described below, and expressed in Escherichia coli.

In order to use as primers for amplifying the maleate isomerase gene, a pair of oligonucleotides having the following sequences obtained by adding a recognition sequence for the restriction enzyme XhoI to sequences on 5'-side and 3'-side of the nucleotide sequence shown in SEQ ID NO: 4 respectively were synthesized by using a 394 DNA/RNA synthesizer produced by Applied Biosystems.
(A) 5'-GGG CTC GAG ATG AAA ACC TAC CGC ATC-3' (SEQ ID NO: 9)
(B) 5'-CCC CTC GAG TCA GGC TTG TGG TTT GAC-3' (SEQ ID NO: 10)

In the sequences, A indicates adenine, G indicates guanine, C indicates cytosine, and T indicates thymine.

A PCR process was performed under the following condition by using the primers described above, using a template of the XhoI fragment of about 2 kb containing the maleate isomerase gene obtained in the item (1)-4, and using a DNA thermal cycler produced by Perkin-Elmer and Cetus.

### 〈Composition of reaction solution〉

50 mM KCl
10 mM Tris-HCl (pH 8.4)
1.5 mM MgCl₂
template DNA 5 µl
primer A 0.25 µM
primer B 0.25 µM
each of dNTPs 200 µM
Taq DNA polymerase (produced by Takara Shuzo) 2.5 units

The components listed above were mixed to give a volume of 100 µl.

### 〈PCR process〉

denaturation step: 94 °C, 60 seconds
annealing step: 37 °C, 120 seconds
extension step: 72 °C, 180 seconds

The steps described above constituted 1 cycle, and 30 cycles were performed in the PCR.

A reaction solution (10 µl) after the PCR process was electrophoresed with a 4 % polyacrylamide gel to confirm that only the DNA fragment of about 800 bp was amplified.

Next, the amplified DNA fragment of about 800 bp was treated with the restriction enzyme XhoI to remove unnecessary portions located on the both ends, followed by a treatment for forming blunt ends by using DNA Blunting Kit (produced by Takara Shuzo). The DNA fragment (1 µg) after the treatment was mixed with an expression vector pKK223-3 (produced by Pharmacia) having been digested with a restriction enzyme SmaI and then subjected to a dephosphorylation treatment, to which respective components of 50 mM Tris buffer (pH 7.6), 10 mM dithiothreitol, 1 mM ATP, 10 mM MgCl₂, and T4 DNA ligase (1 unit) were added (concentrations of the respective components were final concentrations), followed by a reaction at 16 °C for 10 hours to ligate the both.

Escherichia coli JM109 (produced by Takara Shuzo) was transformed with a solution obtained by the ligation reaction in accordance with a calcium chloride method [Journal of Molecular Biology, Vol. 53, p. 159 (1970)], which was spread over a medium [Trypton 10 g, yeast extract 5 g, NaCl 5 g, and agar 16 g dissolved in 1 L of distilled water] containing 50 µg/ml of ampicillin. A transformant strain grown on the medium was cultivated at 37 °C for 12 hours by using a liquid medium having the same composition as described above. Next, plasmid DNA was extracted from microbial cells obtained by the cultivation. The plasmid was digested with a restriction enzyme EcoRI, and subjected to polyacrylamide gel electrophoresis and agarose gel electrophoresis respectively to investigate the size of digested fragments. As a result, we succeeded in confirming the existence of a DNA fragment of about 5 kb (4.6 kb + 0.4 kb) containing the plasmid pKK223-3 (1.6 kb) and a fragment of 370 bp. Accordingly, it was revealed that the plasmid obtained by the foregoing operation contained the fragment of the maleate isomerase gene of about 800 bp inserted in a forward direction at a position downstream from a promoter (3'-side). This plasmid was designated as pMI13, and Escherichia coli JM 109 strain (produced by Takara Shuzo) transformed with the plasmid was designated as ECMI13.

### Example 3: Isolation of Maleate Isomerase Gene Originating from Bacillus stearothermophilus MI-101 Strain and Construction of Transformant

### (1) Cloning of DNA fragment containing maleate isomerase gene:

### (1)-1 Purification of maleate isomerase, determination of partial amino acid sequence, and preparation of DNA probe based thereon:

A medium A [100 ml, composition: disodium maleate 5 g, KH₂PO₄ 0.7 g, K₂HPO₄ 1.4 g, NH₄NO₃ 1 g, MgSO₄·7H₂O 0.2 g, biotin 0.2 mg, thiamin hydrochloride 0.2 mg, FeSO₄·7H₂O 20 mg, MnSO₄ 20 mg, distilled water 1 L (adjusted at pH 7.2)] was dispensed and poured into an Erlenmeyer flask having a volume of 500 ml, and the medium was subjected to a sterilization treatment at 120 °C for 20 minutes, to which Bacillus stearothermophilus MI-101 strain was seeded, followed by cultivation at 50 °C for 24 hours with shaking.

Microbial cells were recovered from a culture as described in the item (1)-1 in Example 2, and a crude enzyme solution was obtained in the same manner as described above.

Next, the crude enzyme solution was subjected to ammonium sulfate fractionation (35 to 70 %), followed by dialysis overnight at 4 °C against the phosphate buffer described above. An active fraction of maleate isomerase was obtained from the dialyzed solution in the same manner as the operation described in the item (1)-1 in Example 2.

The purified fraction of maleate isomerase was subjected to electrophoresis in the same manner as described in the item (1)-1 in Example 2 to make separation. Consequently, an equivalent result was obtained. According to the result, it has been assumed that the maleate isomerase has a structure of a dimer having a molecular weight of about 60 kDa comprising subunits having a molecular weight of about 30 kDa.

An amino acid sequence from the N-terminal of the purified isomerase was determined by using an amino acid sequencer (produced by Applied Biosystems, 473A type). A result is shown in SEQ ID NO: 13.

Next, probe DNA's having nucleotide sequences shown in SEQ ID NOs: 14 and 15 deduced from the obtained amino acid sequence were synthesized by using a DNA synthesizer (produced by Applied Biosystems, 394 type).

### (1)-2 Preparation of chromosomal DNA fragment containing maleate isomerase gene:

Bacillus stearothermophilus MI-101 strain was cultivated with shaking at 50 °C by using the medium A described above until a late logarithmic growth phase to collect microbial cells.

A chromosomal DNA solution was obtained from a lysate of the recovered microbial cells in the same manner as described in the item (1)-2 in Example 2.

Next, a restriction enzyme EcoRI (50 U (units)) was added to 90 µl of the chromosomal DNA solution to make a reaction at 37 °C for 1 hour so that complete degradation was achieved. An obtained solution was subjected to agarose gel electrophoresis. DNA was transferred from the agarose gel to a nylon membrane to perform Southern hybridization in accordance with an ordinary method ["Molecular Cloning", Cold Spring Harbor Laboratory Press (1989)] by using the synthetic probes shown in SEQ ID NOs: 14 and 15 with their 5'-terminal phosphate groups radioisotope-labeled [Anal. Biochem., 158, 307-315 (1986)] by using T4 polynucleotide kinase (produced by Takara Shuzo) and [γ-³²P]ATP.

As a result, it was confirmed that a DNA fragment, to which the synthetic probes strongly hybridized, was present at a position of about 4 kb on the nylon membrane.

### (1)-3 Insertion of chromosomal DNA into vector:

A λDNA library of the chromosomal DNA was obtained from the chromosomal DNA solution of Bacillus stearothermophilus MI-101 strain in accordance with the same operation as described in Example 2.

### (1)-4 Selection of objective recombinant DNA:

A phage solution of the λDNA library prepared in the foregoing item (1)-3 (2-5 x 10⁴ pfu; diluted with SM buffer) was mixed with an equivalent amount of a culture of Escherichia coli P2392, which was kept at a temperature of 37 °C for 15 minutes. A λ medium (3 to 4 ml, 1 % Trypton, 0.5 % yeast extract, 0.5 % NaCl, 0.2 % MgSO₄·7H₂O, 0.5 % agar) having been kept at a temperature of 50 °C was added to the mixed solution, which was homogeneously applied to a λ plate (1 % Trypton, 0.5 % NaCl, 0.2 % MgSO₄·7H₂O, 1 % agar), followed by cultivation at 37 °C for 12 to 16 hours.

A nitrocellulose filter was placed on the medium so that plaques formed on the medium were adsorbed to the filter. The filter was treated in the same manner as described in Example 2 to immobilize DNA.

The filter prepared as described above was subjected to plaque hybridization by using the probes shown in SEQ ID NOs: 14 and 15 prepared in the foregoing item (1)-1 in accordance with an ordinary method ["Molecular Cloning", Cold Spring Harbor Laboratory Press (1989)].

As a result, a hybridization-positive plaque for the both probes was selected, from which phage DNA was extracted to confirm an inserted fragment having a length of about 4 kb excised by the restriction enzyme EcoRI by means of agarose gel electrophoresis.

In order to further specify the position of the maleate isomerase gene existing on the obtained DNA (EcoRI-EcoRI) fragment having the length of about 4 kb, the DNA fragment was subcloned into a plasmid pUC118 (produced by Takara Shuzo) as follows.

The operation was performed in the same manner as described in Example 2 except that EcoRI was used as the restriction enzyme to transform Escherichia coli JM109. It was also confirmed that the DNA fragment having the length of about 4 kb was present in the inserted fragment.

A crude enzyme solution was prepared from a transformant in the same manner as described in the item (1)-4 in Example 1. It was confirmed that the transformant had the maleate isomerase activity.

### (2) Determination of nucleotide sequence:

A nucleotide sequence of the DNA (EcoRI-EcoRI) fragment having the length of about 4 kb containing the maleate isomerase gene obtained in the foregoing item (1)-4 was determined in accordance with a dideoxychain termination method [Sanger, F. et al., Proc. Natl. Acad. Sci. USA, Vol. 74, p. 5463 (1977)] by using pUC118 (produced by Takara Shuzo).

According to the existence of an open reading frame in the determined nucleotide sequence, it has been revealed that the maleate isomerase gene has a nucleotide sequence shown in SEQ ID NO: 11 in Sequence Listing described later on and comprises 753 base pairs coding for 251 amino acids. The amino acid sequence is shown in SEQ ID NO: 12.

It has been confirmed that the determined nucleotide sequence contains a sequence corresponding to the entire nucleotide sequence deduced from the amino acid sequence (SEQ ID NO: 13) determined in the foregoing item (1)-1.

### (3) Preparation of expression plasmid and construction of transformant:

A gene region (about 800 bp) coding for maleate isomerase was amplified by the PCR (Polymerase Chain Reaction) method on the basis of the nucleotide sequence of SEQ ID NO: 11 determined in the item (2), which was inserted into an expression vector autonomously replicable in Escherichia coli as described below, and expressed in Escherichia coli.

In order to use as primers for amplifying the maleate isomerase gene, a pair of oligonucleotides having the following sequences obtained by adding recognition sequences for restriction enzymes SmaI and PstI to sequences on 5'-side and 3'-side of the nucleotide sequence shown in SEO ID NO: 11 respectively were synthesized by using a 394 DNA/RNA synthesizer produced by Applied Biosystems.
(C) 5'-TTTCCCGGGATGGCAAAACATTTTCGT-3' (SEQ ID NO: 16)
(D) 5'-CCCCTGCAGTTAATATTTTCCTGACAA-3' (SEQ ID NO: 17)

In the sequences, A indicates adenine, G indicates guanine, C indicates cytosine, and T indicates thymine.

The PCR was performed under the following condition by using the primers described above, using a template of the chromosome of Bacillus stearothermophilus MI-101 strain obtained in the item (1)-2, and using a DNA thermal cycler produced by Perkin-Elmer and Cetus.

### 〈Composition of reaction solution〉

50 mM KCl
10 mM Tris-HCl (pH 8.4)
1.5 mM MgCl₂
template DNA 5 µl
primer C 0.25 µM
primer D 0.25 µM
each of dNTPs 200 µM
Taq DNA polymerase (produced by Takara Shuzo) 2.5 units

The components listed above were mixed to give a volume of 100 µl.

### 〈PCR process〉

denaturation step: 94 °C, 60 seconds
annealing step: 37 °C, 120 seconds
extension step: 72 °C, 180 seconds

The steps described above constituted 1 cycle, and 30 cycles were performed in the PCR.

A reaction solution (10 µl) after the PCR process was electrophoresed with a 4 % polyacrylamide gel to confirm that only the DNA fragment of about 800 bp was amplified.

Next, the amplified DNA fragment of about 800 bp was treated with the restriction enzymes SmaI and PstI to remove unnecessary portions located on the both ends. The DNA fragment (1 µg) after the treatment was mixed with an expression vector pKK223-3 (produced by Pharmacia) having been digested with the restriction enzymes SmaI and PstI, to which respective components of 50 mM Tris buffer (pH 7.6), 10 mM dithiothreitol, 1 mM ATP, 10 mM MgCl₂, and T4 DNA ligase (1 unit) were added (concentrations of the respective components were final concentrations), followed by a reaction at 16 °C for 10 hours to ligate the both.

Escherichia coli JM109 (produced by Takara Shuzo) was transformed with a solution obtained by the ligation reaction in accordance with a calcium chloride method [Journal of Molecular Biology, Vol. 53, p. 159 (1970)], which was spread over a medium (Trypton 10 g, yeast extract 5 g, NaCl 5 g, and agar 16 g dissolved in 1 L of distilled water) containing 50 µg/ml of ampicillin. A transformant strain grown on the medium was cultivated at 37 °C for 12 hours by using a liquid medium having the same composition as described above. Next, plasmid DNA was extracted from microbial cells obtained by the cultivation. The plasmid was digested with the restriction enzymes SmaI and PstI, and subjected to polyacrylamide gel electrophoresis and agarose gel electrophoresis respectively to investigate the size of digested fragments. As a result, we succeeded in confirming the existence of a DNA fragment of about 4.6 kb of the plasmid pKK223-3 and a fragment of about 800 bp of the maleate isomerase gene. Accordingly, it was revealed that the plasmid obtained by the foregoing operation contained the fragment of the maleate isomerase gene of about 800 bp inserted in a forward direction at a position downstream from a promoter (3'-side). This plasmid was designated as pSP101, and Escherichia coli JM 109 strain (produced by Takara Shuzo) transformed with the plasmid was designated as MISP101.

### Example 4: Isolation of Maleate Isomerase Gene Originating from Bacillus stearothermophilus MI-102 Strain and Construction of Transformant

### (1) Cloning of DNA fragment containing maleate isomerase gene:

### (1)-1 Purification of maleate isomerase, determination of partial amino acid sequence, and preparation of DNA probe based thereon:

The operation was performed in the same manner as described in the item (1)-1 in Example 3 by using Bacillus stearothermophilus MI-102 strain. It has been assumed that the maleate isomerase has a structure of a dimer having a molecular weight of about 60 kDa comprising subunits having a molecular weight of about 30 kDa.

An amino acid sequence from the N-terminal of the purified isomerase was determined by using an amino acid sequencer (produced by Applied Biosystems, 473A type). A result is shown in SEQ ID NO: 20.

Next, probe DNA's having nucleotide sequences shown in SEQ ID NOs: 21 and 22 deduced from the obtained amino acid sequence were synthesized by using a DNA synthesizer (produced by Applied Biosystems, 394 type).

### (1)-2 Preparation of chromosomal DNA fragment containing maleate isomerase gene:

A chromosomal DNA solution was obtained from Bacillus stearothermophilus MI-102 strain in accordance with the same operation as described in Example 3.

Next, the operation was performed in the same manner as described in Example 3 except that a restriction enzyme FabI was used, with the use of the synthetic probes shown in SEQ ID NOs: 21 and 22 with their 5'-terminal phosphate groups radioisotope-labeled [Anal. Biochem., 158, 307-315 (1986)]. It was confirmed that a DNA fragment, to which the synthetic probes strongly hybridized, was present at a position of about 2 kb on the nylon membrane.

### (1)-3 Insertion of chromosomal DNA into vector:

A λDNA library of chromosomal DNA was obtained from the chromosomal DNA solution of Bacillus stearothermophilus MI-102 strain in accordance with the same operation as described in Example 2.

### (1)-4 Selection of objective recombinant DNA:

Plaque hybridization was performed in the same manner as described in Example 3 except that a phage solution of the λDNA library obtained in the foregoing item (1)-3 was used, and the sequences shown in SEQ ID NOs: 21 and 22 were used as probes.

As a result, a hybridization-positive plaque for the both probes was selected, from which phage DNA was extracted to confirm an inserted fragment having a length of about 2 kb excised by a restriction enzyme HindIII by means of agarose gel electrophoresis.

Subcloning was performed and the inserted fragment was confirmed as well for the DNA fragment having the length of about 2 kb in the same manner as described in Example 3 except that HindIII was used as the restriction enzyme.

A crude enzyme solution was prepared from a strain obtained by the foregoing subcloning in the same manner as described in the item (1)-4 in Example 1. It was confirmed that the subcloned strain had the maleate isomerase activity.

### (2) Determination of nucleotide sequence:

A nucleotide sequence of the DNA (HindIII-HindIII) fragment having the length of about 2 kb containing the maleate isomerase gene obtained in the foregoing item (1)-4 was determined in the same manner as described in Example 3.

According to the existence of an open reading frame in the determined nucleotide sequence, it has been revealed that the maleate isomerase gene has a nucleotide sequence shown in SEQ ID NO: 18 in Sequence Listing described later on and comprises 753 base pairs coding for 251 amino acids. The amino acid sequence is shown in SEQ ID NO: 19.

It has been confirmed that the determined nucleotide sequence contains a sequence corresponding to the entire nucleotide sequence deduced from the amino acid sequence (SEQ ID NO: 20) determined in the foregoing item (1)-1.

### (3) Preparation of expression plasmid and construction of transformant:

The same operation as described in Example 3 was performed except that primers as shown in SEQ ID NOs: 23 (5'-side) and 24 (3'-side) were used for the sequence shown in SEQ ID NO: 18 determined in the item (2).
(E) 5'-TTTCCCGGGATGGCAAAACATTTTCGT-3' (SEQ ID NO: 23)
(F) 5'-CCCCTGCAGTTAATATTTTCCTGACAA-3' (SEQ ID NO: 24)

An obtained plasmid was designated as pSP102, and Escherichia coli JM 109 strain (produced by Takara Shuzo) transformed with the plasmid was designated as MISP102.

### Example 5: Isolation of Maleate Isomerase Gene Originating from Bacillus brevis MI-103 Strain and Construction of Transformant

### (1) Cloning of DNA fragment containing maleate isomerase gene:

### (1)-1 Purification of maleate isomerase, determination of partial amino acid sequence, and preparation of DNA probe based thereon:

The operation was performed in the same manner as described in the item (1)-1 in Example 3 by using Bacillus brevis MI-103 strain. It has been assumed that the maleate isomerase has a structure of a dimer having a molecular weight of about 60 kDa comprising subunits having a molecular weight of about 30 kDa.

An amino acid sequence from the N-terminal of the purified isomerase was determined by using an amino acid sequencer (produced by Applied Biosystems, 473A type). A result is shown in SEQ ID NO: 27.

Next, probe DNA's having nucleotide sequences shown in SEQ ID NOs: 28 and 29 deduced from the obtained amino acid sequence were synthesized by using a DNA synthesizer (produced by Applied Biosystems, 394 type).

### (1)-2 Preparation of chromosomal DNA fragment containing maleate isomerase gene:

A chromosomal DNA solution was obtained from Bacillus brevis MI-103 strain in accordance with the same operation as described in Example 3.

Next, the operation was performed in the same manner as described in Example 3 except that a restriction enzyme HindIII was used, with the use of the synthetic probes shown in SEQ ID NOs: 28 and 29 with their 5'-terminal phosphate groups radioisotope-labeled [Anal. Biochem., 158, 307-315 (1986)]. It was confirmed that a DNA fragment, to which the synthetic probes strongly hybridized, was present at a position of about 4.5 kb on the nylon membrane.

### (1)-3 Insertion of chromosomal DNA into vector:

A λDNA library of chromosomal DNA was obtained from the chromosomal DNA solution of Bacillus brevis MI-103 strain in accordance with the same operation as described in Example 2.

### (1)-4 Selection of objective recombinant DNA:

Plaque hybridization was performed in the same manner as described in Example 3 except that a phage solution of the λDNA library obtained in the foregoing item (1)-3 was used, and the sequences shown in SEQ ID NOs: 28 and 29 were used as probes.

As a result, a hybridization-positive plaque for the both probes was selected, from which phage DNA was extracted to confirm an inserted fragment having a length of about 4.5 kb excised by a restriction enzyme FabI by means of agarose gel electrophoresis.

Subcloning was performed and the inserted fragment was confirmed as well for the DNA fragment having the length of about 4.5 kb in the same manner as described in Example 3 except that FabI was used as the restriction enzyme.

A crude enzyme solution was prepared from a strain obtained by the foregoing subcloning in the same manner as described in the item (1)-4 in Example 1. It was confirmed that the subcloned strain had the maleate isomerase activity.

### (2) Determination of nucleotide sequence:

A nucleotide sequence of the DNA (FabI-FabI) fragment having the length of about 4.5 kb containing the maleate isomerase gene obtained in the foregoing item (1)-4 was determined in the same manner as described in Example 3.

According to the existence of an open reading frame in the determined nucleotide sequence, it has been revealed that the maleate isomerase gene has a nucleotide sequence shown in SEQ ID NO: 25 in Sequence Listing described later on and comprises 756 base pairs coding for 252 amino acids. The amino acid sequence is shown in SEQ ID NO: 26.

It has been confirmed that the determined nucleotide sequence contains a sequence corresponding to the entire nucleotide sequence deduced from the amino acid sequence (SEQ ID NO: 27) determined in the foregoing item (1)-1.

### (3) Preparation of expression plasmid and construction of transformant:

The same operation as described in Example 3 was performed except that primers as shown in SEQ ID NOs: 30 (5'-side) and 31 (3'-side) were used for the sequence shown in SEQ ID NO: 25 determined in the item (2).
(G) 5'-TTTCCCGGGATGAGCCCAAAAAACTAT-3' (SEQ ID NO: 30)
(F) 5'-CCCCTGCAGTTAAAACGAACCGGATAA-3' (SEQ ID NO: 31)

An obtained plasmid was designated as pSP103, and Escherichia coli JM 109 strain (produced by Takara Shuzo) transformed with the plasmid was designated as MISP103.

### Example 6: Isolation of Maleate Isomerase Gene Originating from Bacillus stearothermophilus MI-105 Strainand Construction of Transformant

### (1) Cloning of DNA fragment containing maleate isomerase gene:

### (1)-1 Purification of maleate isomerase, determination of partial amino acid sequence, and preparation of DNA probe based thereon:

The operation was performed in the same manner as described in the item (1)-1 in Example 3 by using Bacillus stearothermophilus MI-105 strain. It has been assumed that the maleate isomerase has a structure of a dimer having a molecular weight of about 60 kDa comprising subunits having a molecular weight of about 30 kDa.

An amino acid sequence from the N-terminal of the purified isomerase was determined by using an amino acid sequencer (produced by Applied Biosystems, 473A type). A result is shown in SEQ ID NO: 34.

Next, probe DNA's having nucleotide sequences shown in SEQ ID NOs: 35 and 36 deduced from the obtained amino acid sequence were synthesized by using a DNA synthesizer (produced by Applied Biosystems, 394 type).

### (1)-2 Preparation of chromosomal DNA fragment containing maleate isomerase gene:

A chromosomal DNA solution was obtained from Bacillus stearothermophilus MI-105 strain in accordance with the same operation as described in Example 3.

Next, the operation was performed in the same manner as described in Example 3 except for the use of the synthetic probes shown in SEQ ID NOs: 35 and 36 with their 5'-terminal phosphate groups radioisotope-labeled [Anal. Biochem., 158, 307-315 (1986)]. It was confirmed that a DNA fragment, to which the synthetic probes strongly hybridized, was present at a position of about 1.9 kb on the nylon membrane.

### (1)-3 Insertion of chromosomal DNA into vector:

A λDNA library of chromosomal DNA was obtained from the chromosomal DNA solution of Bacillus stearothermophilus MI-105 strain in accordance with the same operation as described in Example 2.

### (1)-4 Selection of objective recombinant DNA:

Plaque hybridization was performed in the same manner as described in Example 3 except that a phage solution of the λDNA library obtained in the foregoing item (1)-3 was used, and the sequences shown in SEQ ID NOs: 35 and 36 were used as probes.

As a result, a hybridization-positive plaque for the both probes was selected, from which phage DNA was extracted to confirm an inserted fragment having a length of about 1.9 kb excised by a restriction enzyme EcoRI by means of agarose gel electrophoresis.

Subcloning was performed and the inserted fragment was confirmed as well for the DNA fragment having the length of about 1.9 kb in the same manner as described in Example 3.

A crude enzyme solution was prepared from a strain obtained by the foregoing subcloning in the same manner as described in the item (1)-4 in Example 1. It was confirmed that the subcloned strain had the maleate isomerase activity.

### (2) Determination of nucleotide sequence:

A nucleotide sequence of the DNA (EcoRI-EcoRI) fragment having the length of about 1.9 kb containing the maleate isomerase gene obtained in the foregoing item (1)-4 was determined in the same manner as described in Example 3.

According to the existence of an open reading frame in the determined nucleotide sequence, it has been revealed that the maleate isomerase gene has a nucleotide sequence shown in SEQ ID NO: 32 in Sequence Listing described later on and comprises 753 base pairs coding for 251 amino acids. The amino acid sequence is shown in SEQ ID NO: 33.

It has been confirmed that the determined nucleotide sequence contains a sequence corresponding to the entire nucleotide sequence deduced from the amino acid sequence (SEQ ID NO: 34) determined in the foregoing item (1)-1.

### (3) Preparation of expression plasmid and construction of transformant

The same operation as described in Example 3 was performed except that primers as shown in SEQ ID NOs: 37 (5'-side) and 38 (3'-side) were used for the sequence shown in SEQ ID NO: 32 determined in the item (2).
(I) 5'-TTTCCCGGGATGGTAAAACACTTTCGT-3' (SEQ ID NO: 37)
(J) 5'-CCCCTGCAGTTAATATTTCCCTGACAA-3' (SEQ ID NO: 38)

An obtained plasmid was designated as pSP105, and Escherichia coli JM 109 strain (produced by Takara Shuzo) transformed with the plasmid was designated as MISP105.

### Example 7: Isolation of Maleate Isomerase Gene Originating from Bacillus stearothermophilus MI-110 Strain and Construction of Transformant

### (1) Cloning of DNA fragment containing maleate isomerase gene:

### (1)-1 Purification of maleate isomerase, determination of partial amino acid sequence, and preparation of DNA probe based thereon:

The operation was performed in the same manner as described in the item (1)-1 in Example 3 by using Bacillus stearothermophilus MI-110 strain. It has been assumed that the maleate isomerase has a structure of a dimer having a molecular weight of about 60 kDa comprising subunits having a molecular weight of about 30 kDa.

An amino acid sequence from the N-terminal of the purified isomerase was determined by using an amino acid sequencer (produced by Applied Biosystems, 473A type). A result is shown in SEQ ID NO: 41.

Next, probe DNA's having nucleotide sequences shown in SEQ ID NOs: 42 and 43 deduced from the obtained amino acid sequence were synthesized by using a DNA synthesizer (produced by Applied Biosystems, 394 type).

### (1)-2 Preparation of chromosomal DNA fragment containing maleate isomerase gene:

A chromosomal DNA solution was obtained from Bacillus stearothermophilus MI-110 strain in accordance with the same operation as described in Example 3.

Next, the operation was performed in the same manner as described in Example 3 except that a restriction enzyme PstI was used, with the use of the synthetic probes shown in SEQ ID NOs: 42 and 43 with their 5'-terminal phosphate groups radioisotope-labeled [Anal. Biochem., 158, 307-315 (1986)]. It was confirmed that a DNA fragment, to which the synthetic probes strongly hybridized, was present at a position of about 2.9 kb on the nylon membrane.

### (1)-3 Insertion of chromosomal DNA into vector:

A λDNA library of chromosomal DNA was obtained from the chromosomal DNA solution of Bacillus stearothermophilus MI-110 strain in accordance with the same operation as described in Example 2.

### (1)-4 Selection of objective recombinant DNA:

Plaque hybridization was performed in the same manner as described in Example 3 except that a phage solution of the λDNA library obtained in the foregoing item (1)-3 was used, and the sequences shown in SEQ ID NOs: 42 and 43 were used as probes.

As a result, a hybridization-positive plaque for the both probes was selected, from which phage DNA was extracted to confirm an inserted fragment having a length of about 2.9 kb excised by the restriction enzyme PstI by means of agarose gel electrophoresis.

Subcloning was performed and the inserted fragment was confirmed as well for the DNA fragment having the length of about 2.9 kb in the same manner as described in Example 3 except that PstI was used as the restriction enzyme.

A crude enzyme solution was prepared from a strain obtained by the foregoing subcloning in the same manner as described in the item (1)-4 in Example 1. It was confirmed that the subcloned strain had the maleate isomerase activity.

### (2) Determination of nucleotide sequence:

A nucleotide sequence of the DNA (PstI-PstI) fragment having the length of about 2.9 kb containing the maleate isomerase gene obtained in the foregoing item (1)-4 was determined in the same manner as described in Example 3.

A crude enzyme solution was prepared from the subcloned strain in the same manner as described in the item (1)-4 in Example 1. It was confirmed that the subcloned strain had the maleate isomerase activity.

According to the existence of an open reading frame in the determined nucleotide sequence, it has been revealed that the maleate isomerase gene has a nucleotide sequence shown in SEQ ID NO: 39 in Sequence Listing described later on and comprises 753 base pairs coding for 251 amino acids. The amino acid sequence is shown in SEQ ID NO: 40.

It has been confirmed that the determined nucleotide sequence contains a sequence corresponding to the entire nucleotide sequence deduced from the amino acid sequence (SEQ ID NO: 41) determined in the foregoing item (1)-1.

### (3) Preparation of expression plasmid and construction of transformant:

The same operation as described in Example 3 was performed except that primers as shown in SEQ ID NOs: 44 (5'-side) and 45 (3'-side) were used for the sequence shown in SEQ ID NO: 39 determined in the item (2).
(K) 5'-TTTCCCGGGATGGCAAAACATTTTCGT-3' (SEQ ID NO: 44)
(L) 5'-CCCCTGCAGTTAATATTTTCCTGACAA-3' (SEQ ID NO: 45)

An obtained plasmid was designated as pSP110, and Escherichia coli JM 109 strain (produced by Takara Shuzo) transformed with the plasmid was designated as MISP110.

### Example 8: Isolation of Maleate Isomerase Gene Originating from Serratia marcescens IFO 3736 Strain and Construction of Transformant

### (1) Cloning of DNA fragment containing maleate isomerase gene:

### (1)-1 Purification of maleate isomerase, determination of partial amino acid sequence, and preparation of DNA probe based thereon:

A medium B [100 ml, composition: disodium maleate 5 g, (NH₄)SO₄ 2g, KH₂PO₄ 1.4 g, Na₂HPO₄·12H₂O 3.1 g, MgSO₄·7H₂O 0.25 mg, yeast extract 0.5 g, distilled water 1 L (adjusted at pH 6.0 with NaOH)] was dispensed and poured into an Erlenmeyer flask having a volume of 500 ml, and the medium was subjected to a sterilization treatment at 120 °C for 20 minutes, to which Serratia marcescens IFO 3736 strain was seeded, followed by cultivation at 30 °C for 24 hours with shaking.

The operation of recovery of microbial cells from a culture and the following operations were performed in the same manner as described in the item (1)-1 in Example 3. According to results thus obtained, it has been assumed that the maleate isomerase has a structure of a dimer having a molecular weight of about 60 kDa comprising subunits having a molecular weight of about 30 kDa.

An amino acid sequence from the N-terminal of the purified isomerase was determined by using an amino acid sequencer (produced by Applied Biosystems, 473A type). A result is shown in SEQ ID NO: 48.

Next, probe DNA's having nucleotide sequences shown in SEQ ID NOs: 49 and 50 deduced from the obtained amino acid sequence were synthesized by using a DNA synthesizer (produced by Applied Biosystems, 394 type).

### (1)-2 Preparation of chromosomal DNA fragment containing maleate isomerase gene:

Serratia marcescens IFO 3736 strain was cultivated with shaking at 30 °C by using the medium B described above until a late logarithmic growth phase to collect microbial cells.

Next, the operation was performed in the same manner as described in Example 3 except that a restriction enzyme PstI was used, with the use of the synthetic probes shown in SEQ ID NOs: 49 and 50 with their 5'-terminal phosphate groups radioisotope-labeled [Anal. Biochem., 158, 307-315 (1986)]. It was confirmed that a DNA fragment, to which the synthetic probes strongly hybridized, was present at a position of about 1.9 kb on the nylon membrane.

### (1)-3 Insertion of chromosomal DNA into vector:

A λDNA library of chromosomal DNA was obtained from a chromosomal DNA solution of Serratia marcescens IFO 3736 strain in accordance with the same operation as described in Example 2.

### (1)-4 Selection of objective recombinant DNA:

Plaque hybridization was performed in the same manner as described in Example 3 except that a phage solution of the λDNA library obtained in the foregoing item (1)-3 was used, and the sequences shown in SEQ ID NOs: 49 and 50 were used as probes.

As a result, a hybridization-positive plaque for the both probes was selected, from which phage DNA was extracted to confirm an inserted fragment having a length of about 1.9 kb excised by the restriction enzyme PstI by means of agarose gel electrophoresis.

Subcloning was performed and the inserted fragment was confirmed as well for the DNA fragment having the length of about 1.9 kb in the same manner as described in Example 3 except that PstI was used as the restriction enzyme.

A crude enzyme solution was prepared from a strain obtained by the foregoing subcloning in the same manner as described in the item (1)-4 in Example 1. It was confirmed that the subcloned strain had the maleate isomerase activity.

### (2) Determination of nucleotide sequence:

A nucleotide sequence of the DNA (PstI-PstI) fragment having the length of about 1.9 kb containing the maleate isomerase gene obtained in the foregoing item (1)-4 was determined in the same manner as described in Example 3.

According to the existence of an open reading frame in the determined nucleotide sequence, it has been revealed that the maleate isomerase gene has a nucleotide sequence shown in SEQ ID NO: 46 in Sequence Listing described later on and comprises 750 base pairs coding for 250 amino acids. The amino acid sequence is shown in SEQ ID NO: 47.

It has been confirmed that the determined nucleotide sequence contains a sequence corresponding to the entire nucleotide sequence deduced from the amino acid sequence (SEQ ID NO: 48) determined in the foregoing item (1)-1.

### (3) Preparation of expression plasmid and construction of transformant:

The same operation as described in Example 3 was performed except that primers as shown in SEQ ID NOs: 51 (5'-side) and 52 (3'-side) were used for the sequence shown in SEQ ID NO: 46 determined in the item (2).
(M) 5'-TTTCCCGGGATGAGCAACCACTACCGC-3' (SEQ ID NO: 51)
(N) 5'-CCCCTGCAGTCAATAAGCGCCGGACAG-3' (SEQ ID NO: 52)

An obtained plasmid was designated as pSP3736, and Escherichia coli JM 109 strain (produced by Takara Shuzo) transformed with the plasmid was designated as MISP3736.

### Example 9: Production of Maleate Isomerase and Confirmation of Activity

Each of the MISP-strains constructed in Examples 2 to 8 was seeded to the LB medium (liquid medium from which agar was removed) containing 50 µg/ml of ampicillin, 5 g/L of sodium maleate, and 1 g/L of malonic acid, and aerobically cultivated with shaking at 37 °C for 15 hours. An obtained culture was centrifuged (3,000 x g, 4 °C, 20 minutes) to recover microbial cells which were then washed with a phosphate buffer [composition: 20 mM potassium phosphate buffer (pH 7.2), 0.5 mM dithiothreitol].

Next, the washed microbial cells (0.5 g, wet weight) were suspended in the phosphate buffer (2 ml), and treated with an ultrasonic disrupter (produced by Branson) under cooling with ice to obtain a disrupted preparation of the microbial cells. The disrupted preparation was centrifuged (10,000 x g, 4 °C, 30 minutes) to obtain a supernatant as a crude enzyme solution.

The plasmid pKK223-3 containing no foreign gene was used as a control, from which a transformant and a crude enzyme solution were prepared exactly in the same manner as described above to be used for the following activity measurement.

The maleate isomerase activity was confirmed for the prepared crude enzyme solutions by measuring the decrease in maleic acid as the change in absorbance at 240 nm in the presence of fumarase originating from swine heart (produced by Boehringer Mannheim) in the same manner as described in the foregoing item (1) in Example 1 [K. Otsuka, Agric. Biol. Chem., Vol. 25, p. 726 (1961)]. As a result, the absorbance at 240 nm decreased only for the crude enzyme solutions prepared from the respective MISP-strains. Thus we succeeded in detecting the maleate isomerase activity.

### Example 10: Isomerization Reaction of Maleic Acid by Transformant Containing Maleate Isomerase

In the same manner as described in Example 9, each of the MISP101 to MISP3736 strains constructed in Examples 2 to 8 was seeded to the LB medium (liquid medium from which agar was removed) containing 50 µg/ml of ampicillin, and aerobically cultivated with shaking at 37 °C for 15 hours. An obtained culture was centrifuged (3,000 x g, 4 °C, 20 minutes) to recover microbial cells which were then washed with 0.9 % (w/v) NaCl.

Next, the washed microbial cells (0.1 g, wet weight) were suspended in 0.9 % (w/v) NaCl (1 ml), to which a reaction solution [1 ml, composition: sodium maleate 84 g/L, Triton X-100 1 g/L] was added, followed by a reaction at 30 °C for 1 hour with shaking. The reaction solution after the reaction was centrifuged (3,000 x g, 4 °C, 20 minutes) to obtain a supernatant which was subjected to high-performance liquid chromatography analysis (LC-5A, produced by Shimadzu) by using a column for organic acid analysis (SCR-101H column produced by Shimadzu) and a UV detector (210 nm). As a result, we succeeded in detecting a peak of fumaric acid in addition to a peak of maleic acid as the raw material. It was also confirmed that the reaction brought about a decrease in maleic acid as the raw material.

According to the results described above, it has been confirmed that maleic acid is isomerized into fumaric acid by the action of the maleate isomerase contained in each of the transformants.

### Reference Examples: Introduction of Site-Directed Mutagenesis into Maleate Isomerase Originating from Alcaligenes faecalis IFO 13111, and Evaluation of Enzyme Activity of Mutant Enzyme

### Reference Example 1: Site-Directed Mutagenesis of Maleate Isomerase Originating from Alcaligenes faecalis IFO 13111, and Construction of Transformant Harboring Mutant Enzyme Gene

### (A) Design of PCR primers

Site-directed mutation was introduced into maleate isomerase originating from Alcaligenes faecalis IFO 13111 by using a recombinant PCR method (R. Higuchi et al., Recombinant PCR in "PCR Protocols: A Guide to Methods and Applications", p. 177, Academic Press, 1990) to construct mutant enzymes C64S, C80S, and C198S in which three cysteine residues located at 64th, 80th, and 198th positions of the maleate isomerase were substituted with serine respectively (see Fig. 3).

In order to substitute the three cysteine residues located at 64th, 80th, and 198th positions of the maleate isomerase originating from Alcaligenes faecalis IFO 13111 with serine, TGC codons (coding for Cys) located at 190-192th, 238-240th, and 592-594th positions in the nucleotide sequence of the maleate isomerase gene originating from Alcaligenes faecalis IFO 13111 were converted into TCC codons (coding for Ser) by means of site-directed mutagenesis. Sequences of oligo DNA's used for recombinant PCR were designed as follows.

It is noted that CS1F, CS2F, and CS3F are primers of a sense strand, while CS1R, CS2R, and CS3R are primers of an antisense strand having sequences complementary thereto. SMA13 was designed to add a SmaI recognition sequence to a forward end of the 5'-terminal sequence, and HAD13 was designed to add a HindIII recognition sequence to a forward end of a sequence complementary to the 3'-terminal sequence.
Sequence CS1F:
TCGCCAGCGA CCGCTCCGCA GTTGAACTGA (SEQ ID NO: 53 corresponding to 176 to 205 of SEQ ID NO: 4)
Sequence CS1R:
TCAGTTCAAC TGCGGAGCGG TCGCTGGCGA (SEQ ID NO: 54)
Sequence CS2F:
ATGGCCTACG CTTCCCTGGT TGCCATCATG (SEQ ID NO: 55 corresponding to 226 to 255 of SEQ ID NO: 4)
Sequence CS2R:
CATGATGGCA ACCAGGGAAG CGTAGGCCAT (SEQ ID NO: 56)
Sequence CS3F:
TTCTGTCCGC TTCCGTGCAG ATGCCTTCCC (SEQ ID NO: 57 corresponding to 581 to 610 of SEQ ID NO: 4)
Sequence CS3R:
GGGAAGGCAT CTGCACGGAA GCGGACAGAA (SEQ ID NO: 58)
Sequence SMA13
CCCCCCCGGG ATGAAAACCT ACCGCATCGG (SEQ ID NO: 59 corresponding to 1 to 20 of SEQ ID NO: 4)
Sequence HND13
TTTTAAGCTT TCAGGCTTGT GGTTTGACCC (SEQ ID NO: 60 corresponding to 743 to 762 of SEQ ID NO: 4)

### (B) 1st PCR process

It was expected to obtain PCR reaction products having sizes shown in Table 5 respectively in conformity with amplification of portions of the maleate isomerase gene on the chromosome originating from Alcaligenes faecalis IFO 13111, upon practice of PCR in accordance with combinations shown in Table 5 by using the primers described in the foregoing item (A) and using the chromosome originating from Alcaligenes faecalis IFO 13111 prepared in the item (1)-2 in Example 2 as a template.

**Table 5**

| Number | Primer 1 | Primer 2 | Fragment size |
|---|---|---|---|
| CS0 | SMA13 | HND13 | about 770 bp |
| CS1A | SMA13 | CS1R | about 200 bp |
| CS1B | CS1F | HND13 | about 580 bp |
| CS2A | SMA13 | CS2R | about 250 bp |
| CS2B | CS2F | HND13 | about 530 bp |
| CS3A | SMA13 | CS3R | about 610 bp |
| CS3B | CS3F | HND13 | about 170 bp |

The PCR process was actually performed under the following condition by using a DNA thermal cycler produced by Perkin-Elmer and Cetus.

### 〈Reaction solution〉

50 mM KCl
10 mM Tris-HCl (pH 8.4)
1.5 mM MgCl₂
template DNA 5 µl
each of primers prepared in the item (A) 0.25 µM
each of dNTPs 200 µM
Taq DNA polymerase (produced by Takara Shuzo) 2.5 units

The components listed above were mixed to give a volume of 100 µl.

### 〈PCR cycle〉

denaturation step: 94 °C, 60 seconds
annealing step: 37 °C, 120 seconds
extension step: 72 °C, 180 seconds

The steps described above constituted 1 cycle, and 30 cycles were performed.

A reaction solution (10 µl) produced as described above was electrophoresed with a 2 % agarose gel. As a result, DNA fragments having the sizes shown in Table 5 were detected.

### (2) 2nd PCR process

Next, it was expected to obtain PCR reaction products having sizes shown in Table 6 respectively by performing amplification in a state of ligation of the entire maleate isomerase gene on the chromosome originating from Alcaligenes faecalis IFO 13111, upon practice of PCR in accordance with combinations shown in Table 6 by using the primers SMA13 and HND13 described above and using the PCR reaction products prepared in the foregoing item (B) as templates. The condition for PCR was in conformity with that described in the foregoing item (B). However, the template DNA used was obtained by excising the PCR product produced in the foregoing item (B) from the gel after the electrophoresis, and purifying it with GENECLEAN KIT (produced by BIO 101, INC.).

**Table 6**

| Number | Template 1 | Template 2 | Fragment size |
|---|---|---|---|
| CS1 | CS1A | CS1B | about 770 bp |
| CS2 | CS2A | CS2B | about 770 bp |
| CS3 | CS3A | CS3B | about 770 bp |

A reaction solution (10 µl) produced as described above was electrophoresed with a 2 % agarose gel. As a result, DNA fragments each having the size of about 770 bp were detected.

### (D) Cloning of amplified fragment

The PCR product CS0 obtained in the foregoing item (B), the PCR products CS1, CS2, and CS3 obtained in the foregoing item (C), and a high-performance expression vector pKK223-3 (commercially available from Pharmacia) for Escherichia coli were digested with restriction enzymes SmaI and HindIII respectively, and then they were mixed with each other, to which respective components of 50 mM Tris buffer (pH 7.6), 10 mM dithiothreitol, 1 mM ATP, 10 mM MgCl₂, and T4 DNA ligase (1 unit) were added (concentrations of the respective components were final concentrations), followed by a reaction at 4 °C for 15 hours to achieve ligation.

Escherichia coli JM109 (produced by Takara Shuzo) was transformed by using an obtained plasmid mixture solution in accordance with a calcium chloride method [Journal of Molecular Biology, 53, 159 (1970)], which was spread over a medium [Trypton 10 g, yeast extract 5 g, NaCl 5 g, and agar 16 g dissolved in 1 L of distilled water] containing 50 mg of ampicillin.

Respective strains grown on the medium were cultivated in a liquid medium in accordance with an ordinary method. Plasmid DNA's were extracted from cultures. The plasmids were digested with the restriction enzymes SmaI and HindIII to confirm inserted fragments. As a result, inserted fragments having lengths of about 760 bp corresponding to CS0, CS1, CS2, and CS3 were confirmed in addition to a DNA fragment having a length of 4.6 kb corresponding to the plasmid pKK223-3. The respective plasmids were designated as pKK-CS0, pKK-CS1, pKK-CS2, and pKK-CS3. Escherichia coli JM109 strains containing the plasmids were designated as Escherichia coli ECS0, ECS1, ECS2, and ECS3 respectively.

### Reference Example 2: Evaluation of Enzyme Activity of Mutant Enzyme of Maleate Isomerase Originating from Alcaligenes faecalis IFO 13111

Escherichia coli ECS0, ECS1, ECS2, and ECS3 strains constructed in Reference Example 1 were seeded to an LB medium (Trypton 10 g, yeast extract 5 g, NaCl 5 g) containing 50 µg/ml of ampicillin respectively, and they were aerobically cultivated at 30 °C for 15 hours with shaking. Obtained cultures were centrifuged (3,000 x g, 4 °C, 20 minutes) to recover microbial cells which were then washed with 0.9 % (w/v) NaCl.

The plasmid pKK223-3 containing no foreign gene was used as a control, from which a transformant and washed microbial cells were prepared exactly in the same manner as described above to be used for the following activity measurement.

Next, the washed cells (0.1 g, wet weight) were suspended in 0.9 % (w/v) NaCl (1 ml), to which a reaction solution [1 ml, composition: sodium maleate 84 g/L, Triton X-100 1 g/L] was added, followed by a reaction at 45 °C for 1 hour with shaking. The reaction solution after the reaction was centrifuged (3,000 x g, 4 °C, 20 minutes) to obtain a supernatant which was subjected to high-performance liquid chromatography analysis (LC-5A, produced by Shimadzu) by using a column for organic acid (SCR-101H column produced by Shimadzu) and a UV detector (210 nm).

Table 7 shows results of measurement of the activity of maleate isomerase on the basis of the decrease in maleic acid detected by HPLC. The activity is represented by a relative value calculated by regarding the activity of the ECS0 strain (strain harboring the wild type enzyme gene) as 100.

**Table 6**

| | |
|---|---|
| ECS0 | 100 |
| ECS1 | 90 |
| ECS2 | <5 |
| ECS3 | <5 |
| pKK223-3/JM109 | <5 |

According to the results, it has been demonstrated that the 80th and 198th cysteine residues are important for the activity of maleate isomerase originating from Alcaligenes faecalis IFO 13111.

### Industrial Applicability

According to the present invention, there are provided DNA coding for maleate isomerase, a recombinant vector comprising the DNA, and a transformant harboring the recombinant vector.

Maleate isomerase can be produced in a considerable amount by using the transformant harboring the maleate isomerase gene of the present invention. Fumaric acid can be efficiently produced from maleic acid by utilizing microbial cells of the transformant or the maleate isomerase prepared from the microbial cells.

SEQUENCE LISTING

(1) GENERAL INFORMATION:
(i) APPLICANT:
(ii) TITLE OF INVENTION: DNA Coding for Maleate Isomerase
and Utilization Thereof
(iii) NUMBER OF SEQUENCES: 60
(iv) CORRESPONDENCE ADDRESS:
(A) ADDRESSEE:
(B) STREET:
(C) CITY:
(E) COUNTRY:
(F) ZIP:
(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: FastSEQ Version 1.5
(vi) CURRENT APPLICATION DATA:
(A) APPLICATION NUMBER:
(B) FILING DATE:
(C) CLASSIFICATION:
(vii) PRIOR APPLICATION DATA:
(A) APPLICATION NUMBER:
(B) FILING DATE:
(viii) ATTORNEY/AGENT INFORMATION:
(A) NAME:
(B) REGISTRATION NUMBER:
(ix) TELECOMMUNICATION INFORMATION:
(A) TELEPHONE:
(B) TELEFAX:

(2) INFORMATION FOR SEQ ID NO:1:
(i) SEQUENCE CHARACTERISTICS:
(A) LENGTH: 8 amino acids
(B) TYPE: amino acid
(ii) MOLECULE TYPE: protein
(ix) FEATURE:
(D) OTHER INFORMATION: Xaa=Val or Ile
(xi) SEQUENCE DESCRIPTION: SEQ ID NO:1:
Tyr Ala Cys Leu Xaa Ala Ile Met
1 5 8

(2) INFORMATION FOR SEQ ID NO:2:

(i) SEQUENCE CHARACTERISTICS:
(A) LENGTH: 14 amino acids
(B) TYPE: amino acid
(ii) MOLECULE TYPE: protein
(ix) FEATURE:
(D) OTHER INFORMATION: Xaa=Val or Ile
(xi) SEQUENCE DESCRIPTION: SEQ ID NO:2:
Asp Ala Xaa Xaa Leu Ser Ala Cys Val Gln Met Pro Ser Leu
1 5 10 14

(2) INFORMATION FOR SEQ ID NO:3:
(i) SEQUENCE CHARACTERISTICS:
(A) LENGTH: 255 amino acids
(B) TYPE: amino acid
(C) STRANDEDNESS: single
(D) TOPOLOG: linear
(ii) MOLECULE TYPE: protein
(xi) SEQUENCE DESCRIPTION: SEQ ID NO:3:
Met Xaa Xaa Xaa Xaa Xaa Arg Xaa Gly Xaa Ile Val Pro Ser Ser Asn
1               5                   10                  15
Thr Thr Met Glu Thr Glu Ile Pro Ala Met Leu Xaa Xaa Arg Xaa Xaa
            20                  25                  30
Xaa Xaa Xaa Xaa Glu Xaa Phe Thr Phe His Ser Ser Arg Met Arg Met
        35                  40                  45
Xaa His Val Xar Xas Glu Glu Leu Xaa Xaa Met Asp Xaa Xaa Ser Asp
50 55 60
Arg Cys Ala Xaa Glu Leu Ser Asp Ala Arg Xaa Xaa Val Xaa Xaa Tyr
65 70 75 80
Ala Cys Leu Xaa Ala Ile Met Xaa Xaa Gly Xaa Gly Tyr His Xaa Xaa
85 90 95
Ser Xaa Xaa Arg Xaa Xaa Xaa Xaa Xaa Xaa Xaa Asn Xaa Xaa Xaa Xaa
100 105 110
Pro Val Xaa Ser Ser Ala Gly Ala Leu Xaa Xaa Xaa Xaa Xaa Xaa Xaa
115 120 125
Gly Xaa Lys Xaa Xaa Xaa Xaa Xaa Xaa Pro Tyr Met Lys Pro Leu Thr
130 135 140
Xaa Xaa Val Xaa Xaa Tyr Xaa Xaa Xaa Xaa Gly Ile Glu Val Xaa Xaa
145 150 155 160
Xaa Xaa Xaa Leu Glu Xaa Xaa Asp Asn Leu Xaa Val Gly Xaa Xaa Xaa
165 170 175
Pro Xaa Xaa Leu Xaa Xaa Xaa Xaa Xaa Xaa Xaa Xaa Xaa Xaa Xaa Xaa
180 185 190
Asp Ala Xaa Xaa Leu Ser Ala Cys Val Gln Met Pro Ser Leu Xaa Ala
195 200 205
Xaa Xaa Xaa Xaa Xaa Xaa Gln Xaa Xaa Xaa Pro Val Xbz Xaa Ala Ala

210 215 220
Xaa Xaa Thr Xbo Tyr Xaa Xaa Leu Xaa Xaa Leu Xaa Leu Xaa Xaa Xaa
225 230 235 240
Val Pro Xeq Ala Gly Xer Xbz Leu Ser Gly Xaa Xaa Xaa Xaa Xaa
245 250 255

(2) INFORMATION FOR SEQ ID NO:4:
(i) SEQUENCE CHARACTERISTICS:
(A) LENGTH: 759 base pairs
(B) TYPE: nucleic acid
(C) STRANDEDNESS: double
(D) TOPOLOGY: linear
(ii) MOLECULE TYPE: Genomic DNA
(vi) ORIGINAL SOURCE:
(A) ORGANISM: Alcaligenes faecalis
(B) STRAIN: IFO13111
(ix) FEATURE:
(A) NAME/KEY: CDS
(B) LOCATION: 1..759
(C) IDENTIFICATION METHOD: by experiment
(xi) SEQUENCE DESCRIPTION: SEQ ID NO:4:
ATG AAA ACC TAC CGC ATC GGT CAG ATC GTC CCC AGC TCC AAC ACC ACC 48
Met Lys Thr Tyr Arg Ile Gly Gln Ile Val Pro Ser Ser Asn Thr Thr
1               5                   10                  15
ATG GAA ACC GAG ATT CCT GCC ATG TTG CAG GCT CGT TAC GCC GAG TTC 96
Met Glu Thr Glu Ile Pro Ala Met Leu Gln Ala Arg Tyr Ala Glu Phe
            20                  25                  30
CCT GAG GAG CGC TTC ACC TTC CAC TCC TCG CGC ATG CGC ATG ATG CAC 144
Pro Glu Glu Arg Phe Thr Phe His Ser Ser Arg Met Arg Met Met His
        35                  40                  45
GTG AAC CCC GAA GAG CTG AAA GCC ATG GAT ATC GCC AGC GAC CGC TGC 192
Val Asn Pro Glu Glu Leu Lys Ala Met Asp Ile Ala Ser Asp Arg Cys
    50                  55                  60
GCA GTT GAA CTG AGC GAC GCC CGT ATG AGC GTG ATG GCC TAC GCT TGC 240
Ala Val Glu Leu Ser Asp Ala Arg Met Ser Val Met Ala Tyr Ala Cys
65                  70                  75                  80
CTG GTT GCC ATC ATG GCC CAA GGC GAC GGC TAC CAC CGC GTC TCG CAA 288
Leu Val Ala Ile Met Ala Gln Gly Asp Gly Tyr His Arg Val Ser Gln
                85                  90                  95
GCC CGT CTG CAA AAC ACC GTT AAA GAA AAC GGC GTG GAA ATT CCT GTG 336
Ala Arg Leu Gln Asn Thr Val Lys Glu Asn Gly Val Glu Ile Pro Val
            100                 105                 110
CTG AGC TCG GCC GGT GCC CTG GTC GAT ACG CTG AAA GAA TTC GGC TAC 384
Leu Ser Ser Ala Gly Ala Leu Val Asp Thr Leu Lys Glu Phe Gly Tyr
        115                 120                 125

AAG AAA GTC TCC ATC ATC ACC CCT TAC ATG AAA CCG CTG ACC AAG CGC 432
Lys Lys Val Ser Ile Ile Thr Pro Tyr Met Lys Pro Leu Thr Lys Arg
    130                 135                 140
GTG GCT GAC TAC ATC GAA GCT GAA GGC ATC GAA GTT CAA GAC TCC ATC 480
Val Ala Asp Tyr Ile Glu Ala Glu Gly Ile Glu Val Gln Asp Ser Ile
145                 150                 155                 160
AGC CTG GAA GTG TCG GAC AAC CTG GAA GTG GGC CTG CTG AAC CCG GAA 528
Ser Leu Glu Val Ser Asp Asn Leu Glu Val Gly Leu Leu Asn Pro Glu
                165                 170                 175
AAC CTG CTG GAG CAC GTC AAA CGC CTG AAC CAC GAC GGC GTG GAC GCA 576
Asn Leu Leu Glu His Val Lys Arg Leu Asn His Asp Gly Val Asp Ala
            180                 185                 190
GTT ATT CTG TCC GCT TGC GTG CAG ATG CCT TCC CTG CCT GCT ATC CAG 624
Val Ile Leu Ser Ala Cys Val Gln Met Pro Ser Leu Pro Ala Ile Gln
        195                 200                 205
CGC GCC CAA GAC CAG ATC GGC AAA CCC GTT CTG TCC GCC GCT GTT TGG 672
Arg Ala Gln Asp Gln Ile Gly Lys Pro Val Leu Ser Ala Ala Val Trp
    210                 215                 220
ACC GTG TAC CAA ATG CTC AAG AAC CTG GGT CTG GAA ACC CGC GTT CCT 720
Thr Val Tyr Gln Met Leu Lys Asn Leu Gly Leu Glu Thr Arg Val Pro
225                 230                 235                 240
AAC GCT GGG CAC ATC CTG TCT GGG GTC AAA CCA CAA GCC TGA 762
Asn Ala Gly His Ile Leu Ser Gly Val Lys Pro Gln Ala
                245                 250

(2) INFORMATION FOR SEQ ID NO:5:
(i) SEQUENCE CHARACTERISTICS:
(A) LENGTH: 253 amino acids
(B) TYPE: amino acid
(C) STRANDEDNESS: single
(D) TOPOLOGY: linear
(ii) MOLECULE TYPE: protein
(xi) SEQUENCE DESCRIPTION: SEQ ID NO:5:
Met Lys Thr Tyr Arg Ile Gly Gln Ile Val Pro Ser Ser Asn Thr Thr
1               5                   10                  15
Met Glu Thr Glu Ile Pro Ala Met Leu Gln Ala Arg Tyr Ala Glu Phe
            20                  25                  30
Pro Glu Glu Arg Phe Thr Phe His Ser Ser Arg Met Arg Met Met His
        35                  40                  45
Val Asn Pro Glu Glu Leu Lys Ala Met Asp Ile Ala Ser Asp Arg Cys
    50                  55                  60
Ala Val Glu Leu Ser Asp Ala Arg Met Ser Val Met Ala Tyr Ala Cys
65                  70                  75                  80
Leu Val Ala Ile Met Ala Gln Gly Asp Gly Tyr His Arg Val Ser Gln
                85                  90                  95

Ala Arg Leu Gln Asn Thr Val Lys Glu Asn Gly Val Glu Ile Pro Val
            100                 105                 110
Leu Ser Ser Ala Gly Ala Leu Val Asp Thr Leu Lys Glu Phe Gly Tyr
        115                 120                 125
Lys Lys Val Ser Ile Ile Thr Pro Tyr Met Lys Pro Leu Thr Lys Arg
    130                 135                 140
Val Ala Asp Tyr Ile Glu Ala Glu Gly Ile Glu Val Gln Asp Ser Ile
145                 150                 155                 160
Ser Leu Glu Val Ser Asp Asn Leu Glu Val Gly Leu Leu Asn Pro Glu
                165                 170                 175
Asn Leu Leu Glu His Val Lys Arg Leu Asn His Asp Gly Val Asp Ala
            180                 185                 190
Val Ile Leu Ser Ala Cys Val Gln Met Pro Ser Leu Pro Ala Ile Gln
        195                 200                 205
Arg Ala Gln Asp Gln Ile Gly Lys Pro Val Leu Ser Ala Ala Val Trp
    210                 215                 220
Thr Val Tyr Gln Met Leu Lys Asn Leu Gly Leu Glu Thr Arg Val Pro
225                 230                 235                 240
Asn Ala Gly His Ile Leu Ser Gly Val Lys Pro Gln Ala
                245                 250

(2) INFORMATION FOR SEQ ID NO:6:
(i) SEQUENCE CHARACTERISTICS:
(A) LENGTH: 28 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:6:
Lys Thr Tyr Arg Ile Gly Gln Ile Val Pro Ser Ser Asn Thr Thr Met
1               5                   10                  15
Glu Thr Glu Ile Pro Ala Met Leu Gln Ala Arg Tyr
            20                  25

(2) INFORMATION FOR SEQ ID NO:7:
(i) SEQUENCE CHARACTERISTICS:
(A) LENGTH: 32 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(ix) FEATURE:
(D) OTHER INFORMATION: N=inosine
(xi) SEQUENCE DESCRIPTION: SEQ ID NO:7:

ATGAARACNT AYCGNATYGG NCARATYGTN CC                                32

(2) INFORMATION FOR SEQ ID NO:8:
    (i) SEQUENCE CHARACTERISTICS:
        (A) LENGTH: 33 bases
        (B) TYPE: nucleic acid
        (C) STRANDEDNESS: single
        (D) TOPOLOGY: linear
    (ii) MOLECULE TYPE: other nucleic acid
        (A) DESCRIPTION:   /desc = "synthetic DNA"
    (ix) FEATURE:
        (D) OTHER INFORMATION: N=inosine
    (xi) SEQUENCE DESCRIPTION: SEQ ID NO:8:
CARACNACNA TGGARACNGA RATYCCNGCN ATG                               33

(2) INFORMATION FOR SEQ ID NO:9:
    (i) SEQUENCE CHARACTERISTICS:
        (A) LENGTH: 27 bases
        (B) TYPE: nucleic acid
        (C) STRANDEDNESS: single
        (D) TOPOLOGY: linear
    (ii) MOLECULE TYPE: other nucleic acid
        (A) DESCRIPTION:   /desc = "synthetic DNA"
    (xi) SEQUENCE DESCRIPTION: SEQ ID NO:9:
GGGCTCGAGA TGAAAACCTA CCGCATC                                      27

(2) INFORMATION FOR SEQ ID NO:10:
    (i) SEQUENCE CHARACTERISTICS:
        (A) LENGTH: 27 bases
        (B) TYPE: nucleic acid
        (C) STRANDEDNESS: single
        (D) TOPOLOGY: linear
    (ii) MOLECULE TYPE: other nucleic acid
        (A) DESCRIPTION:   /desc = "synthetic DNA"
    (xi) SEQUENCE DESCRIPTION: SEQ ID NO:10:
CCCCTCGAGT CAGGCTTGTG GTTTGAC                                      27

(2) INFORMATION FOR SEQ ID NO:11:
    (i) SEQUENCE CHARACTERISTICS:
        (A) LENGTH: 756 bases
        (B) TYPE: nucleic acid
        (C) STRANDEDNESS: double
        (D) TOPOLOGY: linear
    (ii) MOLECULE TYPE: Genomic DNA
    (vi) ORIGINAL SOURCE:

(A) ORGANISM: Bacillus stearothermophilus
(B) STRAIN: MI-101
(ix) FEATURE:
(A) NAME/KEY: CDS
(B) LOCATION: 1..753
(C) IDENTIFICATION METHOD: by experiment
(xi) SEQUENCE DESCRIPTION: SEQ ID NO:11:
ATG GCA AAA CAT TTT CGT ATC GGC ATG ATT GTA CCA AGC TCA AAT ACG 48
Met Ala Lys His Phe Arg Ile Gly Met Ile Val Pro Ser Ser Asn Thr
1               5                   10                  15
ACA ATG GAA ACG GAA ATT CCA GCG ATG CTG CAA TCG AGA ATG AAG GAA 96
Thr Met Glu Thr Glu Ile Pro Ala Met Leu Gln Ser Arg Met Lys Glu
            20                  25                  30
ATC CCT GAG GAA ACG TTT ACG TTT CAT TCC AGC CGC ATG CGC ATG ATG 144
Ile Pro Glu Glu Thr Phe Thr Phe His Ser Ser Arg Met Arg Met Met
        35                  40                  45
CAC GTA ACG AAA GAA GAG CTG AAG AAA ATG GAT GAG GAA AGC GAC CGT 192
His Val Thr Lys Glu Glu Leu Lys Lys Met Asp Glu Glu Ser Asp Arg
    50                  55                  60
TGT GCT ATT GAA CTG TCT GAT GCC CGC TGT GAC GTT CTC GCC TAT GCA 240
Cys Ala Ile Glu Leu Ser Asp Ala Arg Cys Asp Val Leu Ala Tyr Ala
65                  70                  75                  80
TGC CTT GTT GCA ATT ATG TGC CAG GGG CCG GGA TAC CAT GAA AAG TCG 288
Cys Leu Val Ala Ile Met Cys Gln Gly Pro Gly Tyr His Glu Lys Ser
                85                  90                  95
GAA GCC CGC TTG ACC AGC CTA ACG GCT CAA AAC GGt GGT GCG GCG CCT 336
Glu Ala Arg Leu Thr Ser Leu Thr Ala Gln Asn Gly Gly Ala Ala Pro
            100                 105                 110
GTG ATC AGC AGC GCC GGA GCT CTA ATT GAC GGC ATC CGC ACA TTA GGA 384
Val Ile Ser Ser Ala Gly Ala Leu Ile Asp Gly Ile Arg Thr Leu Gly
        115                 120                 125
GCC AAA AAA ATC GCT ttA ATT GCT CCT TAT ATG AAA CCG TTG ACA AAC 432
Ala Lys Lys Ile Ala Leu Ile Ala Pro Tyr Met Lys Pro Leu Thr Asn
    130                 135                 140
CAA GTC ATT GAG TAC ATC ACG GCA AGC GGA ATT GAA GTG ACC GAT TCC 480
Gln Val Ile Glu Tyr Ile Thr Ala Ser Gly Ile Glu Val Thr Asp Ser
145                 150                 155                 160
ATT AGT CTG GAG ATT CCT GAT AAC CTA GAA GTT GGC AGA CAA GAT CCG 528
Ile Ser Leu Glu Ile Pro Asp Asn Leu Glu Val Gly Arg Gln Asp Pro
                165                 170                 175
ATG CGG CTA ATT GAA ATC GTG AAA AAT CTC GAC GTT AGC AAC GCC GAT 576
Met Arg Leu Ile Glu Ile Val Lys Asn Leu Asp Val Ser Asn Ala Asp
            180                 185                 190
GCT GTT GTG CTG TCA GCT TGT GTG CAA ATG CCT TCG TTG CCG GCG ATT 624
Ala Val Val Leu Ser Ala Cys Val Gln Met Pro Ser Leu Pro Ala Ile

195                 200                 205
CAA AAG GTC CAG GAT CAG ATT GGC CTG CCT GTT CTT TCG GCT GCC ACA     672
Gln Lys Val Gln Asp Gln Ile Gly Leu Pro Val Leu Ser Ala Ala Thr
    210                 215                 220
TCC ACC GTC TAC AAA ATT CTC AAG AGC CTG AAT CTG AAG ACT TAT GTT     720
Ser Thr Val Tyr Lys Ile Leu Lys Ser Leu Asn Leu Lys Thr Tyr Val
225                 230                 235                 240
CCT AAT GCA GGG AGC TTG TTG TCA GGA AAA TAT TAA                     756
Pro Asn Ala Gly Ser Leu Leu Ser Gly Lys Tyr
                245                 250

(2) INFORMATION FOR SEQ ID NO:12:
     (i) SEQUENCE CHARACTERISTICS:
          (A) LENGTH: 251 amino acids
          (B) TYPE: amino acid
          (C) STRANDEDNESS: single
          (D) TOPOLOGY: linear
    (ii) MOLECULE TYPE: protein
    (xi) SEQUENCE DESCRIPTION: SEQ ID NO:12:
Met Ala Lys His Phe Arg Ile Gly Met Ile Val Pro Ser Ser Asn Thr
  1               5                  10                  15
Thr Met Glu Thr Glu Ile Pro Ala Met Leu Gln Ser Arg Met Lys Glu
             20                  25                  30
Ile Pro Glu Glu Thr Phe Thr Phe His Ser Ser Arg Met Arg Met Met
         35                  40                  45
His Val Thr Lys Glu Glu Leu Lys Lys Met Asp Glu Glu Ser Asp Arg
     50                  55                  60
Cys Ala Ile Glu Leu Ser Asp Ala Arg Cys Asp Val Leu Ala Tyr Ala
 65                  70                  75                  80
Cys Leu Val Ala Ile Met Cys Gln Gly Pro Gly Tyr His Glu Lys Ser
                 85                  90                  95
Glu Ala Arg Leu Thr Ser Leu Thr Ala Gln Asn Gly Gly Ala Ala Pro
            100                 105                 110
Val Ile Ser Ser Ala Gly Ala Leu Ile Asp Gly Ile Arg Thr Leu Gly
        115                 120                 125
Ala Lys Lys Ile Ala Leu Ile Ala Pro Tyr Met Lys Pro Leu Thr Asn
    130                 135                 140
Gln Val Ile Glu Tyr Ile Thr Ala Ser Gly Ile Glu Val Thr Asp Ser
145                 150                 155                 160
Ile Ser Leu Glu Ile Pro Asp Asn Leu Glu Val Gly Arg Gln Asp Pro
                165                 170                 175
Met Arg Leu Ile Glu Ile Val Lys Asn Leu Asp Val Ser Asn Ala Asp
            180                 185                 190
Ala Val Val Leu Ser Ala Cys Val Gln Met Pro Ser Leu Pro Ala Ile
        195                 200                 205

Gln Lys Val Gln Asp Gln Ile Gly Leu Pro Val Leu Ser Ala Ala Thr
    210                 215                 220
Ser Thr Val Tyr Lys Ile Leu Lys Ser Leu Asn Leu Lys Thr Tyr Val
225                 230                 235                 240
Pro Asn Ala Gly Ser Leu Leu Ser Gly Lys Tyr
                245                 250

(2) INFORMATION FOR SEQ ID NO:13:
        (i) SEQUENCE CHARACTERISTICS:
              (A) LENGTH: 24 amino acids
              (B) TYPE: amino acid
              (C) STRANDEDNESS: single
              (D) TOPOLOGY: linear
       (ii) MOLECULE TYPE: peptide
       (xi) SEQUENCE DESCRIPTION: SEQ ID NO:13:
Ala Lys His Phe Arg Ile Gly Met Ile Val Pro Ser Ser Asn Thr Thr
  1               5                  10                  15
Met Glu Thr Glu Ile Pro Ala Met
              20              24

(2) INFORMATION FOR SEQ ID NO:14:
      (i) SEQUENCE CHARACTERISTICS:
            (A) LENGTH: 27 bases
            (B) TYPE: nucleic acid
            (C) STRANDEDNESS: single
            (D) TOPOLOGY: linear
     (ii) MOLECULE TYPE: other nucleic acid
            (A) DESCRIPTION:   /desc = "synthetic DNA"
     (ix) FEATURE:
            (D) OTHER INFORMATION: N=inosine
     (xi) SEQUENCE DESCRIPTION: SEQ ID NO:14:
GCNAARCATT TYCGYATYGG NATGATYGTN CC                                    32

(2) INFORMATION FOR SEQ ID NO:15:
      (i) SEQUENCE CHARACTERISTICS:
            (A) LENGTH: 33 bases
            (B) TYPE: nucleic acid
            (C) STRANDEDNESS: single
            (D) TOPOLOGY: linear
     (ii) MOLECULE TYPE: other nucleic acid
            (A) DESCRIPTION:   /desc = "synthetic DNA"
     (ix) FEATURE:
            (D) OTHER INFORMATION: N=inosine
     (xi) SEQUENCE DESCRIPTION: SEQ ID NO:15:
AAYACNACNA TGGARACNGA RATYCCWGCN ATG                                   33

(2) INFORMATION FOR SEQ ID NO:16:
(i) SEQUENCE CHARACTERISTICS:
(A) LENGTH: 27 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:16:
TTTCCCGGGA TGGCAAAACA TTTTCGT 27

(2) INFORMATION FOR SEQ ID NO:17:
(i) SEQUENCE CHARACTERISTICS:
(A) LENGTH: 27 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:17:
CCCCTGCAGTTAATATTTTCCTGACAA 27

(2) INFORMATION FOR SEQ ID NO:18:
(i) SEQUENCE CHARACTERISTICS:
(A) LENGTH: 756 bases
(B) TYPE: nucleic acid
(C) STRANDEDNESS: double
(D) TOPOLOGY: linear
(ii) MOLECULE TYPE: Genomic DNA
(vi) ORIGINAL SOURCE:
(A) ORGANISM: Bacillus stearothermophilus
(B) STRAIN: MI-102
(ix) FEATURE:
(A) NAME/KEY: CDS
(B) LOCATION: 1..753
(C) IDENTIFICATION METHOD: by experiment
(xi) SEQUENCE DESCRIPTION: SEQ ID NO:18:
ATG GCA AAA CAT TTT CGT ATC GGC ATG ATT GTA CCA AGC TCA AAT ACG 48
Met Ala Lys His Phe Arg Ile Gly Met Ile Val Pro Ser Ser Asn Thr
1               5                   10                  15
ACA ATG GAA ACG GAA ATT CCA GCG ATG CTG CAA TCG AGA ATG AAG GAA 96
Thr Met Glu Thr Glu Ile Pro Ala Met Leu Gln Ser Arg Met Lys Glu
            20                  25                  30
ATC CCT GAG GAA ACG TTT ACG TTT CAT TCC AGC CGC ATG CGC ATG ATG 144
Ile Pro Glu Glu Thr Phe Thr Phe His Ser Ser Arg Met Arg Met Met

        35                  40                  45
CAC GTG ACG AAA GAA GAG CTG AAG AAA ATG GAT GAG GAA AGC GAC CGT 192
His Val Thr Lys Glu Glu Leu Lys Lys Met Asp Glu Glu Ser Asp Arg
    50                  55                  60
TGT GCT ATT GAA CTG TCT GAT GCC CGC TGT GAC GTT CTC GCC TAT GCA 240
Cys Ala Ile Glu Leu Ser Asp Ala Arg Cys Asp Val Leu Ala Tyr Ala
65                  70                  75                  80
TGC CTT GTT GCA ATT ATG TGC CAG GGG CCG GGA TAC CAT GAA AAG TCG 288
Cys Leu Val Ala Ile Met Cys Gln Gly Pro Gly Tyr His Glu Lys Ser
                85                  90                  95
GAA GCC CGC TTG GCC AGC CTA ACG GCT CAA AAC GGC GGT GCA GCT CCT 336
Glu Ala Arg Leu Ala Ser Leu Thr Ala Gln Asn Gly Gly Ala Ala Pro
            100                 105                 110
GTG ATC AGT AGC GCC GGA GCT CTA ATT GAC GGC ATC CGC ACA TTA GGA 384
Val Ile Ser Ser Ala Gly Ala Leu Ile Asp Gly Ile Arg Thr Leu Gly
        115                 120                 125
GCC AAA AAA ATC GCT TTA ATT GCT CCT TAT ATG AAA CCG CTG ACA AAC 432
Ala Lys Lys Ile Ala Leu Ile Ala Pro Tyr Met Lys Pro Leu Thr Asn
    130                 135                 140
CAA GTC ATT GAG TAC ATC ACG GCA AGC GGA ATT GAA GTG ACC GAT TCC 480
Gln Val Ile Glu Tyr Ile Thr Ala Ser Gly Ile Glu Val Thr Asp Ser
145                 150                 155                 160
ATT AGT CTG GAG ATT CCT GAT AAC CTA GAA GTT GGC AGA CAA GAT CCG 528
Ile Ser Leu Glu Ile Pro Asp Asn Leu Glu Val Gly Arg Gln Asp Pro
                165                 170                 175
ATG CGG CTG ATT GAA ATC GTG AAA AAT CTC GAC GTT AGC AAC GCC GAT 576
Met Arg Leu Ile Glu Ile Val Lys Asn Leu Asp Val Ser Asn Ala Asp
            180                 185                 190
GCT GTT GTG CTG TCA GCT TGT GTG CAA ATG CCT TCG TTG CCG GCG ATT 624
Ala Val Val Leu Ser Ala Cys Val Gln Met Pro Ser Leu Pro Ala Ile
        195                 200                 205
CAA AAG GTT CAG GAT CAG CTT GGC CTG CCT GTT CTT TCG GCT GCC ACA 672
Gln Lys Val Gln Asp Gln Leu Gly Leu Pro Val Leu Ser Ala Ala Thr
    210                 215                 220
TCC ACC GTC TAC AAA ATT CTC AAG AGC CTG AAT CTG AAG ACT TAT GTT 720
Ser Thr Val Tyr Lys Ile Leu Lys Ser Leu Asn Leu Lys Thr Tyr Val
225                 230                 235                 240
CCT AAT GCA GGG AGC TTG TTG TCA GGA AAA TAT TAA 756
Pro Asn Ala Gly Ser Leu Leu Ser Gly Lys Tyr
                245                 250

(2) INFORMATION FOR SEQ ID NO:19:
(i) SEQUENCE CHARACTERISTICS:
(A) LENGTH: 251 amino acids
(B) TYPE: amino acid

(C) STRANDEDNESS: single
(D) TOPOLOGY: linear
(ii) MOLECULE TYPE: protein
(xi) SEQUENCE DESCRIPTION: SEQ ID NO:19:
Met Ala Lys His Phe Arg Ile Gly Met Ile Val Pro Ser Ser Asn Thr
1               5                   10                  15
Thr Met Glu Thr Glu Ile Pro Ala Met Leu Gln Ser Arg Met Lys Glu
            20                  25                  30
Ile Pro Glu Glu Thr Phe Thr Phe His Ser Ser Arg Met Arg Met Met
        35                  40                  45
His Val Thr Lys Glu Glu Leu Lys Lys Met Asp Glu Glu Ser Asp Arg
    50                  55                  60
Cys Ala Ile Glu Leu Ser Asp Ala Arg Cys Asp Val Leu Ala Tyr Ala
65                  70                  75                  80
Cys Leu Val Ala Ile Met Cys Gln Gly Pro Gly Tyr His Glu Lys Ser
                85                  90                  95
Glu Ala Arg Leu Ala Ser Leu Thr Ala Gln Asn Gly Gly Ala Ala Pro
            100                 105                 110
Val Ile Ser Ser Ala Gly Ala Leu Ile Asp Gly Ile Arg Thr Leu Gly
        115                 120                 125
Ala Lys Lys Ile Ala Leu Ile Ala Pro Tyr Met Lys Pro Leu Thr Asn
    130                 135                 140
Gln Val Ile Glu Tyr Ile Thr Ala Ser Gly Ile Glu Val Thr Asp Ser
145                 150                 155                 160
Ile Ser Leu Glu Ile Pro Asp Asn Leu Glu Val Gly Arg Gln Asp Pro
                165                 170                 175
Met Arg Leu Ile Glu Ile Val Lys Asn Leu Asp Val Ser Asn Ala Asp
            180                 185                 190
Ala Val Val Leu Ser Ala Cys Val Gln Met Pro Ser Leu Pro Ala Ile
        195                 200                 205
Gln Lys Val Gln Asp Gln Leu Gly Leu Pro Val Leu Ser Ala Ala Thr
    210                 215                 220
Ser Thr Val Tyr Lys Ile Leu Lys Ser Leu Asn Leu Lys Thr Tyr Val
225                 230                 235                 240
Pro Asn Ala Gly Ser Leu Leu Ser Gly Lys Tyr
                245                 250

(2) INFORMATION FOR SEQ ID NO:20:
(i) SEQUENCE CHARACTERISTICS:
(A) LENGTH: 24 amino acids
(B) TYPE: amino acid
(C) STRANDEDNESS: single
(D) TOPOLOGY: linear
(ii) MOLECULE TYPE: peptide
(xi) SEQUENCE DESCRIPTION: SEQ ID NO:20:

Ala Lys His Phe Arg Ile Gly Met Ile Val Pro Ser Ser Asn Thr Thr
1               5                   10                  15
Met Glu Thr Glu Ile Pro Ala Met
            20              24

(2) INFORMATION FOR SEQ ID NO:21:
(i) SEQUENCE CHARACTERISTICS:
(A) LENGTH: 32 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(ix) FEATURE:
(D) OTHER INFORMATION: N=inosine
(xi) SEQUENCE DESCRIPTION: SEQ ID NO:21:
GCNAARCATT TYCGYATYGG NATGATYGTN CC 32

(2) INFORMATION FOR SEQ ID NO:22:
(i) SEQUENCE CHARACTERISTICS:
(A) LENGTH: 33 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(ix) FEATURE:
(D) OTHER INFORMATION: N=inosine
(xi) SEQUENCE DESCRIPTION: SEQ ID NO:22:
AAYACNACNA TGGARACNGA RATYCCWGCN ATG 33

(2) INFORMATION FOR SEQ ID NO:23:
(i) SEQUENCE CHARACTERISTICS:
(A) LENGTH: 27 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:23:
TTTCCCGGGA TGGCAAAACA TTTTCGT 27

(2) INFORMATION FOR SEQ ID NO:24:
(i) SEQUENCE CHARACTERISTICS:
(A) LENGTH: 27 bases

(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:24:
CCCCTGCAGT TAATATTTTC CTGACAA 27

(2) INFORMATION FOR SEQ ID NO:25:
(i) SEQUENCE CHARACTERISTICS:
(A) LENGTH: 759 base pairs
(B) TYPE: nucleic acid
(C) STRANDEDNESS: double
(D) TOPOLOGY: linear
(ii) MOLECULE TYPE: genomic DNA
(vi) ORIGINAL SOURCE:
(A) ORGANISM: Bacillus brevis
(B) STRAIN: MI-103
(ix) FEATURE:
(A) NAME/KEY: CDS
(B) LOCATION: 1..756
(C) IDENTIFICATION METHOD: by experiment
(xi) SEQUENCE DESCRIPTION: SEQ ID NO:25:
ATG AGC CCA AAA AAC TAT AGA GTA GGT CTG ATT GTC CCT AGC TCC AAT 48
Met Ser Pro Lys Asn Tyr Arg Val Gly Leu Ile Val Pro Ser Ser Asn
1               5                   10                  15
ACC ACG ATG GAA ACG GAA ATC CCA GCC ATG CTA AAC GCG CGT ATG GGA 96
Thr Thr Met Glu Thr Glu Ile Pro Ala Met Leu Asn Ala Arg Met Gly
            20                  25                  30
ATT GAA CCA AAC GAG ATG TTT ACG TTC CAT TCC AGC CGC ATG CGG ATG 144
Ile Glu Pro Asn Glu Met Phe Thr Phe His Ser Ser Arg Met Arg Met
        35                  40                  45
ATG CAC GTG ACC AAG GAA GAA CTG AAG AAA ATG GAT GAA AGC AGC GAC 192
Met His Val Thr Lys Glu Glu Leu Lys Lys Met Asp Glu Ser Ser Asp
    50                  55                  60
CGC TGT GCC GTG GAG CTT TCC GAT GCA CGC TGT GAC GTG CTC GCT TAT 240
Arg Cys Ala Val Glu Leu Ser Asp Ala Arg Cys Asp Val Leu Ala Tyr
65                  70                  75                  80
GCT TGC CTG ATC GCC ATT ATG TCA CAG GGA GCG GGC TAC CAT TGC TTG 288
Ala Cys Leu Ile Ala Ile Met Ser Gln Gly Ala Gly Tyr His Cys Leu
                85                  90                  95
TCG GAA GAA CGC TTG ACC AAA GCG GTC AGA GAC AAC GGC ACT CCT GCA 336
Ser Glu Glu Arg Leu Thr Lys Ala Val Arg Asp Asn Gly Thr Pro Ala
            100                 105                 110
CCA GTG GTC AGC AGT GCC GGA GCT TTA ATA GAA GGA ATT CAA ACG ATA 384

Pro Val Val Ser Ser Ala Gly Ala Leu Ile Glu Gly Ile Gln Thr Ile
        115                 120                 125
GGC GCG AAG AAA GTT GCC ATT ATC ACC CCT TAT ATG AAG CCT CTG ACA 432
Gly Ala Lys Lys Val Ala Ile Ile Thr Pro Tyr Met Lys Pro Leu Thr
    130                 135                 140
GCC ATG GTT GTT GAT TAC TTG AAT CAA AGC GGT ATT GAA GTA ACG GAT 480
Ala Met Val Val Asp Tyr Leu Asn Gln Ser Gly Ile Glu Val Thr Asp
145                 150                 155                 160
TCG ATC AGC TTG GAA GTA TCG GAT AAC TTG GCC GTA GGA AAG CTG GAT 528
Ser Ile Ser Leu Glu Val Ser Asp Asn Leu Ala Val Gly Lys Leu Asp
                165                 170                 175
CCT CAG AAC CTG ATC CAT ATT TCT AAA GAT CTG AAT ATT AGC GGA GCA 576
Pro Gln Asn Leu Ile His Ile Ser Lys Asp Leu Asn Ile Ser Gly Ala
            180                 185                 190
GAT GCA GTC GTA CTT TCC GCA TGT GTA CAA ATG CCT TCG CTG CCG GCC 624
Asp Ala Val Val Leu Ser Ala Cys Val Gln Met Pro Ser Leu Pro Ala
        195                 200                 205
ATT CAA GCC GTG CAG GAT CAA CTG CGG CTT CCG GTG CTT TCC GCT GCG 672
Ile Gln Ala Val Gln Asp Gln Leu Arg Leu Pro Val Leu Ser Ala Ala
    210                 215                 220
ACC TCA ACG GTC TAC AAA ATT CTC AAA AGC TTG AAT TTG AAA CCG GTT 720
Thr Ser Thr Val Tyr Lys Ile Leu Lys Ser Leu Asn Leu Lys Pro Val
225                 230                 235                 240
GTT CCG AAT GCA GGA CAT TTG TTA TCC GGT TCG TTT TAA 759
Val Pro Asn Ala Gly His Leu Leu Ser Gly Ser Phe
                245                 250

(2) INFORMATION FOR SEQ ID NO:26:
(i) SEQUENCE CHARACTERISTICS:
(A) LENGTH: 252 amino acids
(B) TYPE: amino acid
(C) STRANDEDNESS: single
(D) TOPOLOGY: linear
(ii) MOLECULE TYPE: protein
(xi) SEQUENCE DESCRIPTION: SEQ ID NO:26:
Met Ser Pro Lys Asn Tyr Arg Val Gly Leu Ile Val Pro Ser Ser Asn
1               5                   10                  15
Thr Thr Met Glu Thr Glu Ile Pro Ala Met Leu Asn Ala Arg Met Gly
            20                  25                  30
Ile Glu Pro Asn Glu Met Phe Thr Phe His Ser Ser Arg Met Arg Met
        35                  40                  45
Met His Val Thr Lys Glu Glu Leu Lys Lys Met Asp Glu Ser Ser Asp
    50                  55                  60
Arg Cys Ala Val Glu Leu Ser Asp Ala Arg Cys Asp Val Leu Ala Tyr
65                  70                  75                  80

Ala Cys Leu Ile Ala Ile Met Ser Gln Gly Ala Gly Tyr His Cys Leu
                85                  90                  95
Ser Glu Glu Arg Leu Thr Lys Ala Val Arg Asp Asn Gly Thr Pro Ala
            100                 105                 110
Pro Val Val Ser Ser Ala Gly Ala Leu Ile Glu Gly Ile Gln Thr Ile
        115                 120                 125
Gly Ala Lys Lys Val Ala Ile Ile Thr Pro Tyr Met Lys Pro Leu Thr
    130                 135                 140
Ala Met Val Val Asp Tyr Leu Asn Gln Ser Gly Ile Glu Val Thr Asp
145                 150                 155                 160
Ser Ile Ser Leu Glu Val Ser Asp Asn Leu Ala Val Gly Lys Leu Asp
                165                 170                 175
Pro Gln Asn Leu Ile His Ile Ser Lys Asp Leu Asn Ile Ser Gly Ala
            180                 185                 190
Asp Ala Val Val Leu Ser Ala Cys Val Gln Met Pro Ser Leu Pro Ala
        195                 200                 205
Ile Gln Ala Val Gln Asp Gln Leu Arg Leu Pro Val Leu Ser Ala Ala
    210                 215                 220
Thr Ser Thr Val Tyr Lys Ile Leu Lys Ser Leu Asn Leu Lys Pro Val
225                 230                 235                 240
Val Pro Asn Ala Gly His Leu Leu Ser Gly Ser Phe
                245                 250

(2) INFORMATION FOR SEQ ID NO:27:
     (i) SEQUENCE CHARACTERISTICS:
          (A) LENGTH: 25 amino acids
          (B) TYPE: amino acid
          (C) STRANDEDNESS: single
          (D) TOPOLOGY: linear
    (ii) MOLECULE TYPE: peptide
    (xi) SEQUENCE DESCRIPTION: SEQ ID NO:27:
Ser Pro Lys Asn Tyr Arg Val Gly Leu Ile Val Pro Ser Ser Asn Thr
  1               5                  10                  15
Thr Met Glu Thr Glu Ile Pro Ala Met
             20                  25

(2) INFORMATION FOR SEQ ID NO:28:
     (i) SEQUENCE CHARACTERISTICS:
          (A) LENGTH: 32 bases
          (B) TYPE: nucleic acid
          (C) STRANDEDNESS: single
          (D) TOPOLOGY: linear
    (ii) MOLECULE TYPE: other nucleic acid
          (A) DESCRIPTION:   /desc = "synthetic DNA"

(ix) FEATURE:
(D) OTHER INFORMATION: N=inosine
(xi) SEQUENCE DESCRIPTION: SEQ ID NO:28:
CCNAARAAYT AYCGYGTNGG NYTNATYGTN CC 32

(2) INFORMATION FOR SEQ ID NO:29:
(i) SEQUENCE CHARACTERISTICS:
(A) LENGTH: 33 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(ix) FEATURE:
(D) OTHER INFORMATION: N=inosine
(xi) SEQUENCE DESCRIPTION: SEQ ID NO:29:
AANACNACNA TGGARACNGA RATYCCWGCN ATG 33

(2) INFORMATION FOR SEQ ID NO:30:
(i) SEQUENCE CHARACTERISTICS:
(A) LENGTH: 27 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:30:
TTTCCCGGGA TGAGCCCAAA AAACTAT 27

(2) INFORMATION FOR SEQ ID NO:31:
(i) SEQUENCE CHARACTERISTICS:
(A) LENGTH: 27 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:31:
CCCCTGCAGT TAAAACGAAC CGGATAA 27

(2) INFORMATION FOR SEQ ID NO:32:
(i) SEQUENCE CHARACTERISTICS:
(A) LENGTH: 756 base pairs
(B) TYPE: nucleic acid
(C) STRANDEDNESS: double

(D) TOPOLOGY: linear
(ii) MOLECULE TYPE: genomic DNA
(vi) ORIGINAL SOURCE:
(A) ORGANISM: Bacillus stearothermophilus
(B) STRAIN: MI-105
(ix) FEATURE:
(A) NAME/KEY: CDS
(B) LOCATION: 1..753
(C) IDENTIFICATION METHOD: by experiment
(xi) SEQUENCE DESCRIPTION: SEQ ID NO:32:
ATG GTA AAA CAC TTT CGT GTC GGC CTG ATT GTA CCC AGT TCA AAC ACT 48
Met Val Lys His Phe Arg Val Gly Leu Ile Val Pro Ser Ser Asn Thr
1               5                   10                  15
ACA ATG GAA ACA GAG ATT CCA GCC ATG CTC CAA TGG AGA ATG AAT GAA 96
Thr Met Glu Thr Glu Ile Pro Ala Met Leu Gln Trp Arg Met Asn Glu
            20                  25                  30
CTC CCT GAG GAA ACT TTT ACG TTT CAT TCC AGC CGC ATG CGC ATG ATG 144
Leu Pro Glu Glu Thr Phe Thr Phe His Ser Ser Arg Met Arg Met Met
        35                  40                  45
CAT GTA ACT AAA GAA GAG CTG ACG AAA ATG GAT GAG GAA AGC GAC CGT 192
His Val Thr Lys Glu Glu Leu Thr Lys Met Asp Glu Glu Ser Asp Arg
    50                  55                  60
TGT GCT ATT GAA CTG TCC GAT GCC CGC TGT GAC GTT CTT GCC TAT GCA 240
Cys Ala Ile Glu Leu Ser Asp Ala Arg Cys Asp Val Leu Ala Tyr Ala
65                  70                  75                  80
TGT TTG GTT GCG ATT ATG TGC CAG GGA CCG GGA TAT CAT GAA AAG TCG 288
Cys Leu Val Ala Ile Met Cys Gln Gly Pro Gly Tyr His Glu Lys Ser
                85                  90                  95
GAA GCA CGC CTG GCC GAT CTA ACG GCT CAA AAC GGC GGT GCA GCA CCG 336
Glu Ala Arg Leu Ala Asp Leu Thr Ala Gln Asn Gly Gly Ala Ala Pro
            100                 105                 110
GTG ATC AGC AGC GCC GGA GCC TTG ATT GAC GGG ATC CGA ACA TTA GGA 384
Val Ile Ser Ser Ala Gly Ala Leu Ile Asp Gly Ile Arg Thr Leu Gly
        115                 120                 125
GCC AAA AAA ATT GCT TTA ATC GCT CCT TAT ATG AAA CCG CTG ACA AAT 432
Ala Lys Lys Ile Ala Leu Ile Ala Pro Tyr Met Lys Pro Leu Thr Asn
    130                 135                 140
CAG GTC ATC GAG TAC ATT ACG GCG AGC GGA ATT GAA GTG ACA GAT TCG 480
Gln Val Ile Glu Tyr Ile Thr Ala Ser Gly Ile Glu Val Thr Asp Ser
145                 150                 155                 160
ATT AGT CTG GAG ATT CCA GAT AAC CTG GAA GTC GGC AGA CAA GAT CCG 528
Ile Ser Leu Glu Ile Pro Asp Asn Leu Glu Val Gly Arg Gln Asp Pro
                165                 170                 175
ATG CGT CTA ATT GAA ATC GCA AAA AAT CTT GAC ATC AGC AAC GCT GAT 576
Met Arg Leu Ile Glu Ile Ala Lys Asn Leu Asp Ile Ser Asn Ala Asp

            180                 185                 190
GCC GTT GTA CTA TCG GCT TGT GTG CAA ATG CCT TCT CTG CCG GCA ATT 624
Ala Val Val Leu Ser Ala Cys Val Gln Met Pro Ser Leu Pro Ala Ile
        195                 200                 205
CAA AAA GTG CAG GAT CAG ATT GGT TTG CCT GTT CTT TCA GCT GCA ACA 672
Gln Lys Val Gln Asp Gln Ile Gly Leu Pro Val Leu Ser Ala Ala Thr
    210                 215                 220
TCG ACC GTC TAC AAA ATT CTC AAG AGT CTG AAT CTA AAT CCT TAT GTT 720
Ser Thr Val Tyr Lys Ile Leu Lys Ser Leu Asn Leu Asn Pro Tyr Val
225                 230                 235                 240
CCT AAT GCA GGA AGC TTG TTG TCA GGG AAA TAT TAA 756
Pro Asn Ala Gly Ser Leu Leu Ser Gly Lys Tyr
                245                 250

(2) INFORMATION FOR SEQ ID NO:33:
(i) SEQUENCE CHARACTERISTICS:
(A) LENGTH: 251 amino acids
(B) TYPE: amino acid
(C) STRANDEDNESS: single
(D) TOPOLOGY: linear
(ii) MOLECULE TYPE: protein
(xi) SEQUENCE DESCRIPTION: SEQ ID NO:33:
Met Val Lys His Phe Arg Val Gly Leu Ile Val Pro Ser Ser Asn Thr
1               5                   10                  15
Thr Met Glu Thr Glu Ile Pro Ala Met Leu Gln Trp Arg Met Asn Glu
            20                  25                  30
Leu Pro Glu Glu Thr Phe Thr Phe His Ser Ser Arg Met Arg Met Met
        35                  40                  45
His Val Thr Lys Glu Glu Leu Thr Lys Met Asp Glu Glu Ser Asp Arg
    50                  55                  60
Cys Ala Ile Glu Leu Ser Asp Ala Arg Cys Asp Val Leu Ala Tyr Ala
65                  70                  75                  80
Cys Leu Val Ala Ile Met Cys Gln Gly Pro Gly Tyr His Glu Lys Ser
                85                  90                  95
Glu Ala Arg Leu Ala Asp Leu Thr Ala Gln Asn Gly Gly Ala Ala Pro
            100                 105                 110
Val Ile Ser Ser Ala Gly Ala Leu Ile Asp Gly Ile Arg Thr Leu Gly
        115                 120                 125
Ala Lys Lys Ile Ala Leu Ile Ala Pro Tyr Met Lys Pro Leu Thr Asn
    130                 135                 140
Gln Val Ile Glu Tyr Ile Thr Ala Ser Gly Ile Glu Val Thr Asp Ser
145                 150                 155                 160
Ile Ser Leu Glu Ile Pro Asp Asn Leu Glu Val Gly Arg Gln Asp Pro
                165                 170                 175
Met Arg Leu Ile Glu Ile Ala Lys Asn Leu Asp Ile Ser Asn Ala Asp

180                    185                    190
Ala Val Val Leu Ser Ala Cys Val Gln Met Pro Ser Leu Pro Ala Ile
        195                    200                    205
Gln Lys Val Gln Asp Gln Ile Gly Leu Pro Val Leu Ser Ala Ala Thr
    210                    215                    220
Ser Thr Val Tyr Lys Ile Leu Lys Ser Leu Asn Leu Asn Pro Tyr Val
225                    230                    235                    240
Pro Asn Ala Gly Ser Leu Leu Ser Gly Lys Tyr
                245                    250

(2) INFORMATION FOR SEQ ID NO:34:
     (i) SEQUENCE CHARACTERISTICS:
          (A) LENGTH: 24 amino acids
          (B) TYPE: amino acid
          (C) STRANDEDNESS: single
          (D) TOPOLOGY: linear
    (ii) MOLECULE TYPE: peptide
    (xi) SEQUENCE DESCRIPTION: SEQ ID NO:34:
Val Lys His Phe Arg Val Gly Leu Ile Val Pro Ser Ser Asn Thr Thr
  1               5                  10                  15
Met Glu Thr Glu Ile Pro Ala Met
             20                  24

(2) INFORMATION FOR SEQ ID NO:35:
     (i) SEQUENCE CHARACTERISTICS:
          (A) LENGTH: 32 bases
          (B) TYPE: nucleic acid
          (C) STRANDEDNESS: single
          (D) TOPOLOGY: linear
    (ii) MOLECULE TYPE: other nucleic acid
          (A) DESCRIPTION:   /desc = "synthetic DNA"
    (ix) FEATURE:
          (D) OTHER INFORMATION: N=inosine
    (xi) SEQUENCE DESCRIPTION: SEQ ID NO:35:
GCNAARCATT TYCGYGTNGG NCTNATYGTN CC                                    32

(2) INFORMATION FOR SEQ ID NO:36:
     (i) SEQUENCE CHARACTERISTICS:
          (A) LENGTH: 33 bases
          (B) TYPE: nucleic acid
          (C) STRANDEDNESS: single
          (D) TOPOLOGY: linear
    (ii) MOLECULE TYPE: other nucleic acid
          (A) DESCRIPTION:   /desc = "synthetic DNA"
    (ix) FEATURE:

(D) OTHER INFORMATION: N=inosine
(xi) SEQUENCE DESCRIPTION: SEQ ID NO:36:
AANACNACNA TGGARACNGA RATYCCWGCN ATG 33

(2) INFORMATION FOR SEQ ID NO:37:
(i) SEQUENCE CHARACTERISTICS:
(A) LENGTH: 27 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:37:
TTTCCCGGGA TGGTAAAACA CTTTCGT 27

(2) INFORMATION FOR SEQ ID NO:38:
(i) SEQUENCE CHARACTERISTICS:
(A) LENGTH: 27 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:38:
CCCCTGCAGT TAATATTTCC CTGACAA 27

(2) INFORMATION FOR SEQ ID NO:39:
(i) SEQUENCE CHARACTERISTICS:
(A) LENGTH: 756 base pairs
(B) TYPE: nucleic acid
(C) STRANDEDNESS: double
(D) TOPOLOGY: linear
(ii) MOLECULE TYPE: genomic DNA
(vi) ORIGINAL SOURCE:
(A) ORGANISM: Bacillus stearothermophilus
(B) STRAIN: MI-110
(ix) FEATURE:
(A) NAME/KEY: CDS
(B) LOCATION: 1..753
(C) IDENTIFICATION METHOD: by experiment
(xi) SEQUENCE DESCRIPTION: SEQ ID NO:39:
ATG GCA AAA CAT TTT CGT ATC GGC ATG ATT GTA CCA AGC TCA AAT ACG 48
Met Ala Lys His Phe Arg Ile Gly Met Ile Val Pro Ser Ser Asn Thr
1               5                   10                  15
ACA ATG GAA ACG GAA ATT CCA GCC ATG TTG CAA TGG AGA ATG AAG GAA 96

Thr Met Glu Thr Glu Ile Pro Ala Met Leu Gln Trp Arg Met Lys Glu
            20                  25                  30
ATC CCT GAG GAA ACG TTT ACG TTT CAT TCC AGC CGC ATG CGC ATG ATG 144
Ile Pro Glu Glu Thr Phe Thr Phe His Ser Ser Arg Met Arg Met Met
        35                  40                  45
CAC GTA ACG AAA GAA GAA CTG AAG AAA ATG GAT GAG GAA AGC GAC CGC 192
His Val Thr Lys Glu Glu Leu Lys Lys Met Asp Glu Glu Ser Asp Arg
    50                  55                  60
TGT GCT ATT GAG CtA TCC GAT GCC CGC TGT GAC GTC CTC GCC TAT GCA 240
Cys Ala Ile Glu Leu Ser Asp Ala Arg Cys Asp Val Leu Ala Tyr Ala
65                  70                  75                  80
TGC CTT GTC GCA ATT ATG TGC CAG GGA CCG GGA TAC CAC GAA AAA TCG 288
Cys Leu Val Ala Ile Met Cys Gln Gly Pro Gly Tyr His Glu Lys Ser
                85                  90                  95
GAA GCA CGT CGA CTT AGT CTA ACA GCT CAA AAC GGC GGT GCA GCG CCG 336
Glu Ala Arg Arg Leu Ser Leu Thr Ala Gln Asn Gly Gly Ala Ala Pro
            100                 105                 110
GTG ATC AGC AGC GCC GGG GCT CTA ATT GAC GGT ATC CGC ACA TTG GGA 384
Val Ile Ser Ser Ala Gly Ala Leu Ile Asp Gly Ile Arg Thr Leu Gly
        115                 120                 125
GCG AAA AAA ATT GCT TTA ATT GCT CCT TAT ATG AAA CCG CTG ACA AAT 432
Ala Lys Lys Ile Ala Leu Ile Ala Pro Tyr Met Lys Pro Leu Thr Asn
    130                 135                 140
CAG GTC ATT GAG TAC ATC ACG GCA AGC GGA ATT GAA GTG ACA GAT TCA 480
Gln Val Ile Glu Tyr Ile Thr Ala Ser Gly Ile Glu Val Thr Asp Ser
145                 150                 155                 160
ATC AGT CTG GAG ATT CCA GAT AAC CTA GAA GTC GGC AGA CAA GAT CCG 528
Ile Ser Leu Glu Ile Pro Asp Asn Leu Glu Val Gly Arg Gln Asp Pro
                165                 170                 175
ATG CGG CTA ATT GAT ATC GTG AAA AAT CTC GAC GTT AGC AAT GCC GAT 576
Met Arg Leu Ile Asp Ile Val Lys Asn Leu Asp Val Ser Asn Ala Asp
            180                 185                 190
GCT GTC GTA CTG TCG GCT TGC GTT CAA ATG CCT TCT TTG CAA GCA ATT 624
Ala Val Val Leu Ser Ala Cys Val Gln Met Pro Ser Leu Gln Ala Ile
        195                 200                 205
CAA AAG GTC CAG GAT CAG ATT GGA CTT CCT GTT CTT TCG GCT GCC ACA 672
Gln Lys Val Gln Asp Gln Ile Gly Leu Pro Val Leu Ser Ala Ala Thr
    210                 215                 220
TCT ACC GTT TAC AAA ATT CTC AAG AGC CTG AAT TTG AAA CCT TAT GTT 720
Ser Thr Val Tyr Lys Ile Leu Lys Ser Leu Asn Leu Lys Pro Tyr Val
225                 230                 235                 240
CCT AAC GCG GGG AGC TTG TTG TCA GGA AAA TAT TAA 756
Pro Asn Ala Gly Ser Leu Leu Ser Gly Lys Tyr
                245                 250

(2) INFORMATION FOR SEQ ID NO:40:
(i) SEQUENCE CHARACTERISTICS:
(A) LENGTH: 251 amino acids
(B) TYPE: amino acid
(C) STRANDEDNESS: single
(D) TOPOLOGY: linear
(ii) MOLECULE TYPE: protein
(xi) SEQUENCE DESCRIPTION: SEQ ID NO:40:
Met Ala Lys His Phe Arg Ile Gly Met Ile Val Pro Ser Ser Asn Thr
1               5                   10                  15
Thr Met Glu Thr Glu Ile Pro Ala Met Leu Gln Trp Arg Met Lys Glu
            20                  25                  30
Ile Pro Glu Glu Thr Phe Thr Phe His Ser Ser Arg Met Arg Met Met
        35                  40                  45
His Val Thr Lys Glu Glu Leu Lys Lys Met Asp Glu Glu Ser Asp Arg
    50                  55                  60
Cys Ala Ile Glu Leu Ser Asp Ala Arg Cys Asp Val Leu Ala Tyr Ala
65                  70                  75                  80
Cys Leu Val Ala Ile Met Cys Gln Gly Pro Gly Tyr His Glu Lys Ser
                85                  90                  95
Glu Ala Arg Arg Leu Ser Leu Thr Ala Gln Asn Gly Gly Ala Ala Pro
            100                 105                 110
Val Ile Ser Ser Ala Gly Ala Leu Ile Asp Gly Ile Arg Thr Leu Gly
        115                 120                 125
Ala Lys Lys Ile Ala Leu Ile Ala Pro Tyr Met Lys Pro Leu Thr Asn
    130                 135                 140
Gln Val Ile Glu Tyr Ile Thr Ala Ser Gly Ile Glu Val Thr Asp Ser
145                 150                 155                 160
Ile Ser Leu Glu Ile Pro Asp Asn Leu Glu Val Gly Arg Gln Asp Pro
                165                 170                 175
Met Arg Leu Ile Asp Ile Val Lys Asn Leu Asp Val Ser Asn Ala Asp
            180                 185                 190
Ala Val Val Leu Ser Ala Cys Val Gln Met Pro Ser Leu Gln Ala Ile
        195                 200                 205
Gln Lys Val Gln Asp Gln Ile Gly Leu Pro Val Leu Ser Ala Ala Thr
    210                 215                 220
Ser Thr Val Tyr Lys Ile Leu Lys Ser Leu Asn Leu Lys Pro Tyr Val
225                 230                 235                 240
Pro Asn Ala Gly Ser Leu Leu Ser Gly Lys Tyr
                245                 250

(2) INFORMATION FOR SEQ ID NO:41:
(i) SEQUENCE CHARACTERISTICS:
(A) LENGTH: 24 amino acids
(B) TYPE: amino acid

(C) STRANDEDNESS: single
(D) TOPOLOGY: linear
(ii) MOLECULE TYPE: peptide
(xi) SEQUENCE DESCRIPTION: SEQ ID NO:41:
Ala Lys His Phe Arg Ile Gly Met Ile Val Pro Ser Ser Asn Thr Thr
1               5                   10                  15
Met Glu Thr Glu Ile Pro Ala Met
20 24

(2) INFORMATION FOR SEQ ID NO:42:
(i) SEQUENCE CHARACTERISTICS:
(A) LENGTH: 32 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(ix) FEATURE:
(D) OTHER INFORMATION: N=inosine
(xi) SEQUENCE DESCRIPTION: SEQ ID NO:42:
GCNAARCATT TYCGYATYGG NATGATYGTN CC 32

(2) INFORMATION FOR SEQ ID NO:43:
(i) SEQUENCE CHARACTERISTICS:
(A) LENGTH: 33 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(ix) FEATURE:
(D) OTHER INFORMATION: N=inosine
(xi) SEQUENCE DESCRIPTION: SEQ ID NO:43:
AANACNACNA TGGARACNGA RATYCCWGCN ATG 33

(2) INFORMATION FOR SEQ ID NO:44:
(i) SEQUENCE CHARACTERISTICS:
(A) LENGTH: 27 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:44:
TTTCCCGGGA TGGCAAAACA TTTTCGT 27

(2) INFORMATION FOR SEQ ID NO:45:
(i) SEQUENCE CHARACTERISTICS:
(A) LENGTH: 27 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:45:
CCCCTGCAGT TAATATTTTC CTGACAA 27

(2) INFORMATION FOR SEQ ID NO:46:
(i) SEQUENCE CHARACTERISTICS:
(A) LENGTH: 753 base pairs
(B) TYPE: nucleic acid
(C) STRANDEDNESS: double
(D) TOPOLOGY: linear
(ii) MOLECULE TYPE: genomic DNA
(vi) ORIGINAL SOURCE:
(A) ORGANISM: Serratia marcescens
(B) STRAIN: IFO3736
(ix) FEATURE:
(A) NAME/KEY: CDS
(B) LOCATION: 1..753
(C) IDENTIFICATION METHOD: by experiment
(xi) SEQUENCE DESCRIPTION: SEQ ID NO:46:
ATG AGC AAC CAC TAC CGC ATC GGC CAG ATC GTG CCC AGC TCC AAC ACC 48
Met Ser Asn His Tyr Arg Ile Gly Gln Ile Val Pro Ser Ser Asn Thr
1               5                   10                  15
ACG ATG GAA ACC GAG ATC CCG GCG ATG CTG GGC GCG CGC CAG CTG ATA 96
Thr Met Glu Thr Glu Ile Pro Ala Met Leu Gly Ala Arg Gln Leu Ile
            20                  25                  30
CGC CCG GAG CGT TTC ACC TTT CAC TCC AGC CGC ATG CGC ATG AAA CAC 144
Arg Pro Glu Arg Phe Thr Phe His Ser Ser Arg Met Arg Met Lys His
        35                  40                  45
GTC AAT AAA GAA GAA TTG GCG GCG ATG GAC GCC GAG TCC GAT CGC TGC 192
Val Asn Lys Glu Glu Leu Ala Ala Met Asp Ala Glu Ser Asp Arg Cys
    50                  55                  60
GCG CTG GAG CTG TCC GAC GCG CGG GTC GAC GTG CTC GGC TAC GCC TGC 240
Ala Leu Glu Leu Ser Asp Ala Arg Val Asp Val Leu Gly Tyr Ala Cys
65                  70                  75                  80
CTG GTG GCC ATC ATG GCG ATG GGG CTG GGC TAC CAC CGC GAA TCG CAG 288
Leu Val Ala Ile Met Ala Met Gly Leu Gly Tyr His Arg Glu Ser Gln
                85                  90                  95
GCC CGG CTG GCG CAG GTG ACG AAA GAC AAT CAG GCC GCC GCG CCG GTC 336

Ala Arg Leu Ala Gln Val Thr Lys Asp Asn Gln Ala Ala Ala Pro Val
            100                 105                 110
ATC AGC AGC GCC GGC GCG CTG GTC AAC GGC CTG AAG GTG ATC GGC GCC 384
Ile Ser Ser Ala Gly Ala Leu Val Asn Gly Leu Lys Val Ile Gly Ala
        115                 120                 125
AAA CGC ATC GCG CTG GTG GCG CCC TAC ATG AAA CCG CTG ACC CAG CTG 432
Lys Arg Ile Ala Leu Val Ala Pro Tyr Met Lys Pro Leu Thr Gln Leu
    130                 135                 140
GTG GTG GAC TAC ATC CAG CAC GAA GGC ATC GAG GTC AAG GTA TGG CGC 480
Val Val Asp Tyr Ile Gln His Glu Gly Ile Glu Val Lys Val Trp Arg
145                 150                 155                 160
GCG CTG GAG ATC CCG GAC AAC CTC GAC GTC GGC CGG CAC GAT CCG GCC 528
Ala Leu Glu Ile Pro Asp Asn Leu Asp Val Gly Arg His Asp Pro Ala
                165                 170                 175
AGG CTG CCG GGG ATC GTC GCC GAG ATG GAC TTA CGC GAG GTC GAT GCT 576
Arg Leu Pro Gly Ile Val Ala Glu Met Asp Leu Arg Glu Val Asp Ala
            180                 185                 190
ATC GTG CTG TCC GCC TGC GTG CAG ATG CCT TCG CTG CCG GCC GTC CCG 624
Ile Val Leu Ser Ala Cys Val Gln Met Pro Ser Leu Pro Ala Val Pro
        195                 200                 205
ACG GTG GAG GCC CAA ACC GGC AAA CCG GTG ATC ACC GCC GCC ATC GCC 672
Thr Val Glu Ala Gln Thr Gly Lys Pro Val Ile Thr Ala Ala Ile Ala
    210                 215                 220
ACC ACT TAC GCG ATG CTG ACC GCG CTG GAG CTG GAA CCG ATC GTT CCC 720
Thr Thr Tyr Ala Met Leu Thr Ala Leu Glu Leu Glu Pro Ile Val Pro
225                 230                 235                 240
GGC GCC GGC GCC CTG CTG TCC GGC GCT TAT TGA 753
Gly Ala Gly Ala Leu Leu Ser Gly Ala Tyr
                245                 250

(2) INFORMATION FOR SEQ ID NO:47:
(i) SEQUENCE CHARACTERISTICS:
(A) LENGTH: 250 amino acids
(B) TYPE: amino acid
(C) STRANDEDNESS: single
(D) TOPOLOGY: linear
(ii) MOLECULE TYPE: protein
(xi) SEQUENCE DESCRIPTION: SEQ ID NO:47:
Met Ser Asn His Tyr Arg Ile Gly Gln Ile Val Pro Ser Ser Asn Thr
1               5                   10                  15
Thr Met Glu Thr Glu Ile Pro Ala Met Leu Gly Ala Arg Gln Leu Ile
            20                  25                  30
Arg Pro Glu Arg Phe Thr Phe His Ser Ser Arg Met Arg Met Lys His
        35                  40                  45
Val Asn Lys Glu Glu Leu Ala Ala Met Asp Ala Glu Ser Asp Arg Cys

50                  55                  60
Ala Leu Glu Leu Ser Asp Ala Arg Val Asp Val Leu Gly Tyr Ala Cys
65                  70                  75                  80
Leu Val Ala Ile Met Ala Met Gly Leu Gly Tyr His Arg Glu Ser Gln
                85                  90                  95
Ala Arg Leu Ala Gln Val Thr Lys Asp Asn Gln Ala Ala Ala Pro Val
            100                 105                 110
Ile Ser Ser Ala Gly Ala Leu Val Asn Gly Leu Lys Val Ile Gly Ala
        115                 120                 125
Lys Arg Ile Ala Leu Val Ala Pro Tyr Met Lys Pro Leu Thr Gln Leu
    130                 135                 140
Val Val Asp Tyr Ile Gln His Glu Gly Ile Glu Val Lys Val Trp Arg
145                 150                 155                 160
Ala Leu Glu Ile Pro Asp Asn Leu Asp Val Gly Arg His Asp Pro Ala
                165                 170                 175
Arg Leu Pro Gly Ile Val Ala Glu Met Asp Leu Arg Glu Val Asp Ala
            180                 185                 190
Ile Val Leu Ser Ala Cys Val Gln Met Pro Ser Leu Pro Ala Val Pro
        195                 200                 205
Thr Val Glu Ala Gln Thr Gly Lys Pro Val Ile Thr Ala Ala Ile Ala
    210                 215                 220
Thr Thr Tyr Ala Met Leu Thr Ala Leu Glu Leu Glu Pro Ile Val Pro
225                 230                 235                 240
Gly Ala Gly Ala Leu Leu Ser Gly Ala Tyr
                245                 250

(2) INFORMATION FOR SEQ ID NO:48:
     (i) SEQUENCE CHARACTERISTICS:
          (A) LENGTH: 24 amino acids
          (B) TYPE: amino acid
          (C) STRANDEDNESS: single
          (D) TOPOLOGY: linear
    (ii) MOLECULE TYPE: peptide
    (xi) SEQUENCE DESCRIPTION: SEQ ID NO:48:
Ser Asn His Tyr Arg Ile Gly Gln Ile Val Pro Ser Ser Asn Thr  Thr
 1               5                  10                  15
Met Glu Thr Glu Ile Pro Ala Met
            20              24

(2) INFORMATION FOR SEQ ID NO:49:
     (i) SEQUENCE CHARACTERISTICS:
          (A) LENGTH: 29 bases
          (B) TYPE: nucleic acid
          (C) STRANDEDNESS: single
          (D) TOPOLOGY: linear

(ii) MOLECULE TYPE: other nucleic acid
(A) DESCRIPTION: /desc = "synthetic DNA"
(ix) FEATURE:
(D) OTHER INFORMATION: N=inosine
(xi) SEQUENCE DESCRIPTION: SEQ ID NO:49:
AAYCAYTAYC GNATYGGNCA RATYGTNCC 29

(2) INFORMATION FOR SEQ ID NO:50:
(i) SEQUENCE CHARACTERISTICS:
(A) LENGTH: 33 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(ix) FEATURE:
(D) OTHER INFORMATION: N=inosine
(xi) SEQUENCE DESCRIPTION: SEQ ID NO:50:
AAYACNACNA TGGARACNGA RATYCCWGCN ATG 33

(2) INFORMATION FOR SEQ ID NO:51:
(i) SEQUENCE CHARACTERISTICS:
(A) LENGTH: 27 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:51:
TTTCCCGGGA TGAGCAACCA CTACCGC 27

(2) INFORMATION FOR SEQ ID NO:52:
(i) SEQUENCE CHARACTERISTICS:
(A) LENGTH: 27 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:52:
CCCCTGCAGT CAATAAGCGC CGGACAG 27

(2) INFORMATION FOR SEQ ID NO:53:
(i) SEQUENCE CHARACTERISTICS:
(A) LENGTH: 30 bases

(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:53:
TCGCCAGCGA CCGCTCCGCA GTTGAACTGA 30

(2) INFORMATION FOR SEQ ID NO:54:
(i) SEQUENCE CHARACTERISTICS:
(A) LENGTH: 30 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:54:
TCAGTTCAAC TGCGGAGCGG TCGCTGGCGA 30

(2) INFORMATION FOR SEQ ID NO:55:
(i) SEQUENCE CHARACTERISTICS:
(A) LENGTH: 30 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:55:
ATGGCCTACG CTTCCCTGGT TGCCATCATG 30

(2) INFORMATION FOR SEQ ID NO:56:
(i) SEQUENCE CHARACTERISTICS:
(A) LENGTH: 30 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:56:
CATGATGGCA ACCAGGGAAG CGTAGGCCAT 30

(2) INFORMATION FOR SEQ ID NO:57:
(i) SEQUENCE CHARACTERISTICS:
(A) LENGTH: 30 bases
(B) TYPE: nucleic acid

(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:57:
TTCTGTCCGC TTCCGTGCAG ATGCCTTCCC 30

(2) INFORMATION FOR SEQ ID NO:58:
(i) SEQUENCE CHARACTERISTICS:
(A) LENGTH: 30 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:58:
GGGAAGGCAT CTGCACGGAA GCGGACAGAA 30

(2) INFORMATION FOR SEQ ID NO:59:
(i) SEQUENCE CHARACTERISTICS:
(A) LENGTH: 30 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:59:
CCCCCCCGGG ATGAAAACCT ACCGCATCGG 30

(2) INFORMATION FOR SEQ ID NO:60:
(i) SEQUENCE CHARACTERISTICS:
(A) LENGTH: 30 bases
(B) TYPE: nucleic acid
(C) STRANDEDNESS: single
(D) TOPOLOGY: linear
(ii) MOLECULE TYPE: other nucleic acid
(A) DESCRIPTION: /desc = "synthetic DNA"
(xi) SEQUENCE DESCRIPTION: SEQ ID NO:60:
TTTTAAGCTT TCAGGCTTGT GGTTTGACCC 30

## Claims

1. DNA coding for maleate isomerase;
said maleate isomerase comprising at least an amino acid sequence shown in SEQ ID NO: 1 and an amino acid sequence shown in SEQ ID NO: 2, and optionally having substitution, deletion, and insertion of one or more amino acid residues which do not substantially deteriorate an activity to isomerize maleic acid and produce fumaric acid.

2. The DNA according to claim 1, wherein the DNA codes for an amino acid sequence shown in SEQ ID NO: 3.

3. The DNA according to claim 1, wherein the DNA codes for an amino acid sequence shown in SEQ ID NO: 5.

4. The DNA according to claim 3, wherein the DNA has a nucleotide sequence shown in SEQ ID NO: 4 or a nucleotide sequence substantially identical thereto.

5. The DNA according to claim 1, wherein the DNA codes for an amino acid sequence shown in SEQ ID NO: 12.

6. The DNA according to claim 5, wherein the DNA has a nucleotide sequence shown in SEQ ID NO: 11 or a nucleotide sequence substantially identical thereto.

7. The DNA according to claim 1, wherein the DNA codes for an amino acid sequence shown in SEQ ID NO: 19.

8. The DNA according to claim 7, wherein the DNA has a nucleotide sequence shown in SEQ ID NO: 18 or a nucleotide sequence substantially identical thereto.

9. The DNA according to claim 1, wherein the DNA codes for an amino acid sequence shown in SEQ ID NO: 26.

10. The DNA according to claim 9, wherein the DNA has a nucleotide sequence shown in SEQ ID NO: 25 or a nucleotide sequence substantially identical thereto.

11. The DNA according to claim 1, wherein the DNA codes for an amino acid sequence shown in SEQ ID NO: 33.

12. The DNA according to claim 11, wherein the DNA has a nucleotide sequence shown in SEQ ID NO: 32 or a nucleotide sequence substantially identical thereto.

13. The DNA according to claim 1, wherein the DNA codes for an amino acid sequence shown in SEQ ID NO: 40.

14. The DNA according to claim 13, wherein the DNA has a nucleotide sequence shown in SEQ ID NO: 39 or a nucleotide sequence substantially identical thereto.

15. The DNA according to claim 1, wherein the DNA codes for an amino acid sequence shown in SEQ ID NO: 47.

16. The DNA according to claim 15, wherein the DNA has a nucleotide sequence shown in SEQ ID NO: 46 or a nucleotide sequence substantially identical thereto.

17. A recombinant vector comprising the DNA defined in any one of claims 1 to 16 ligated with a vector.

18. A transformant of a microorganism transformed by introducing the DNA defined in any one of claims 1 to 17.

19. A method for producing maleate isomerase comprising the steps of cultivating, in a medium, a transformant obtained by introducing the DNA defined in any one of claims 1 to 17, and collecting maleate isomerase from a culture of the transformant.

20. A method for producing fumaric acid comprising the steps of adding, to an aqueous solution containing maleic acid, a transformant obtained by introducing the DNA defined in any one of claims 1 to 17, or a crude fraction or a purified enzyme of maleate isomerase collected from a culture of the transformant, and isomerizing maleic acid to produce fumaric acid.
